(19) 

Europäisches
Patentamt

European
Patent Office

Office européen
des brevets

(11) **EP 4 513 497 A2**

(12) **EUROPEAN PATENT APPLICATION**

(43) Date of publication:
**26.02.2025   Bulletin 2025/09**

(21) Application number: **24210956.9**

(22) Date of filing: **19.02.2019**

(51) International Patent Classification (IPC):
*G16B 30/10* (2019.01)

(52) Cooperative Patent Classification (CPC):
**G16B 20/20; G16B 30/10**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.02.2018   US 201862710348 P**

(62) Document number(s) of the earlier application(s) in
accordance with Art. 76 EPC:
**19712061.1 / 3 753 021**

(71) Applicant: **Illumina, Inc.
San Diego, CA 92122 (US)**

(72) Inventor: **OJARD, Eric Jon
San Diego, 92122 (US)**

(74) Representative: **Fish & Richardson P.C.
Highlight Business Towers
Mies-van-der-Rohe-Straße 8
80807 München (DE)**

Remarks:
•This application was filed on 05-11-2024 as a
divisional application to the application mentioned
under INID code 62.
•Claims filed after the date of filing of the application /
after the date of receipt of the divisional application
(Rule 68(4) EPC).

(54) **SYSTEMS AND METHODS FOR CORRELATED ERROR EVENT MITIGATION FOR VARIANT
CALLING**

(57)     Methods, systems, and apparatus, including
computer programs for improving the accuracy of a variant call by accounting for indications of correlated error
events. In one aspect, a method may include actions of
accessing a pileup of sequence reads aligned to a first
region of a reference genome, obtaining information
describing one or more characteristics of each of the
plurality of reads of the pileup, providing one or more
inputs to a probability model describing the one or more
characteristics of the plurality of reads of the pileup,
wherein the probability model is configured to determine
a score, for each hypothesis of one or more hypotheses
selected based on the one or more inputs, that indicates
whether each hypothesis is true, obtaining output infor-
mation for each of the one or more hypotheses, and
determining, based on the obtained output information,
a likelihood that a true variant exists at the first position.

EP 4 513 497 A2

FIG. 1

**Description**

**CROSS-REFERENCE TO RELATED APPLICATIONS**

This application claims the benefit of U.S. Provisional Application No. 62/710,348, filed on February 16, 2018, and titled "Methods, Devices, and Systems for Performing Foreign Read Detection and Burst Error Detection," the disclosure of which is incorporated herein by reference in its entirety.

**BACKGROUND**

A nucleic acid sequencer is an instrument that is configured to automate the process of sequencing nucleic acids such as deoxyribonucleic acid (DNA) or ribonucleic acid (RNA). Nucleic acid sequencing is a process of determining an order of nucleotides in a genetic sequence.

The nucleic acid sequencer is configured to receive a nucleic acid sample and generate output data, referred to as one or more "reads," that represents an order of nucleotides in the nucleic acid sample. The nucleotides in a DNA sample can include one or more of guanine (G), cytosine (C), adenine (A), and thymine (T) in any combination. The nucleotides in an RNA sample can include one or more of G, C, A, and uracil (U) in any combination in any combination.

The DNA reads generated by the DNA sequencer can be mapped and aligned to a known DNA sequence of a reference genome. Once mapped and aligned to a reference genome, the mapped and aligned sequence of reads can be analyzed in view of the reference genome to identify potential variations that exist between the mapped and aligned sequence of reads and the reference genome.

**SUMMARY**

Aspects of the present disclosure are directed towards methods, systems, and apparatus, including computer programs, for accounting for correlated error events that are problematic for variant callers used to determine whether an alternative (hereinafter "alt") allele identified in a pileup of mapped and aligned sequence reads is a true variant.

In accordance with one innovative aspect of the present disclosure, a method for improving the accuracy of a variant call by accounting for indications of correlated error events is disclosed. In some implementations, the method may include actions of methods that include accessing, by one or more computers and from one or more memory devices, a pileup of a plurality of sequence reads aligned to a first region of a reference genome, obtaining, by the one or more computers, information describing one or more characteristics of each of the plurality of reads of the pileup corresponding to a first position of the reference genome, providing, by the one or more computers and based on the obtained information, one or more inputs to a probability model describing the one or more characteristics of the plurality of reads of the pileup, wherein the probability model is configured to determine a score, for each hypothesis of one or more hypotheses selected based on the one or more inputs, that indicates whether the hypothesis is true, obtaining, by the one or more computers, output information for each of the one or more hypotheses, wherein the output information for each of the one or more hypotheses is (i) generated by the probability model based on the probability model's processing of the one or more inputs to the probability model describing the one or more characteristics of the respective reads of the pileup and (ii) indicative of a score that indicates whether the hypothesis is true, and determining, by the one or more computers and based on the obtained output information generated by the probability model for each of the plurality of hypotheses, a likelihood that a true variant exists at the first position.

Other version include corresponding systems, apparatus, and computer programs to perform the actions of methods defined by instructions encoded on computer readable storage devices.

These and other versions may optionally include one or more of the following features. For instance, in some implementations, determining, by the one or more computers and based on the obtained output information generated by the probability model for each of the plurality of hypotheses, a likelihood that a true variant exists at the first position can include determining, by the one or more computers, a collective score that is based on the output information generated by the probability model for each of the plurality of hypothesis, wherein the collective score indicates a likelihood that the true variant exists, determining, by the one or more computers, whether the score generated by the collective score satisfies a predetermined threshold, and based on determining, by the one or more computers, that the collective score satisfies the predetermined threshold, adding information to a VCF file indicating that a true variant exists at the first position.

In some implementations, the information indicating that a true variant exists at the first position can include information identifying (i) the first position, (ii) a candidate alt allele at the first position, (iii) the collective score.

In some implementations, the information describing the one or more characteristics of the respective reads can include information describing (i) a mapping quality score for each sequence read in the pileup at the first position and (ii) a read-allele score for each sequence read in the pileup at the first position for each candidate allele at the first position.

In some implementations, the read-allele score for each read of the pileup at the first position is based on an output produced by a P-HMM model, for each of the reads at the first position, which indicates a probability of observing a read $r_i$ given a particular candidate allele $Gm,\emptyset$

In some implementations, the output information can include first output information for a first hypothesis of the one or more hypotheses that includes a likelihood that the sequence reads at the first position indicate the occurrence of a homozygous reference with a foreign allele that matches the alt, and second output information for a second hypothesis of the one or more hypotheses that includes a likelihood that the sequence reads at the first position indicate the occurrence of a homozygous alt with a foreign allele that matches a reference allele.

In some implementations, the information describing the one or more characteristics of the respective sequence reads can include information describing (i) a read orientation for each sequence read of the pileup at the first position, (ii) a position of each base at the first position within each sequence read of the pileup at the first position with reference to the 5' end of the sequence read, (iii) a read-allele score for each sequence read of the plurality of sequence reads for each candidate allele at the reference position, and (iv) a base quality score for each read for the base at the first position.

In some implementations, the read-allele score for each sequence read of the pileup at the first position is based on an output produced by a P-HMM model, for each of the sequence reads at the first position, that indicates a probability of observing a sequence read $r_i$ given a particular candidate allele $G_{m,\phi}$.

In some implementations, the information describing the one or more characteristics of the respective sequence reads can include information describing (i) a read orientation for each sequence read of the pileup at the first position, (ii) a position of each base at the first position within each sequence read of the pileup at the first position with reference to the 5' end of the sequence read, and (iii) a read-allele score for each sequence read of the plurality of sequence reads for each candidate allele at the reference position.

In some implementations, the output information can includes first output information for a first hypothesis of the one or more hypotheses that can include a likelihood that the sequence reads at the first position indicate the occurrence of a homozygous reference with a sequencing error that matches the alt allele, and second output information for a second hypothesis of the one or more hypotheses that includes a likelihood that the sequence reads at the first position indicate the occurrence of a homozygous alt with a sequencing error that matches the reference allele.

In some implementations, the information describing the one or more characteristics of the respective sequence reads can include information describing (i) a read orientation for each sequence read of the pileup at the first position, (ii) a position of each base at the first position such as position "0" 142 within each sequence read with reference to the 5' end of the sequence read, (iii) a mapping quality score for each sequence read of the pileup at the first position, (iv) a read-allele score for each sequence read of the plurality of reads for each candidate allele at the reference position, and (v) a base quality score for each read for the base aligned at the first position.

In some implementations, the read-allele score for each sequence read of the pileup at the first position is based on an output produced by a P-HMM model, for each of the sequence reads at the first position, that indicates a probability of observing a read $r_i$ given a particular candidate allele $G_{m,\phi}$.

In some implementations, the output information can include a first likelihood that the sequence reads at the first position indicate the occurrence of a homozygous reference with a foreign allele that matches the alt, a second likelihood that the sequence reads at the first position indicate the occurrence of a homozygous alt with a foreign allele that matches a reference allele, a third output information for a first hypothesis of the one or more hypotheses that includes a likelihood that the sequence reads at the first position indicate the occurrence of a homozygous reference with a sequencing error that matches the alt allele, and a fourth output information for a second hypothesis of the one or more hypotheses that includes a likelihood that the sequence reads at the first position indicate the occurrence of a homozygous alt with a sequencing error that matches the reference allele.

In some implementations, the one or more memory devices received the pileup of aligned sequence reads from a Field Programmable Gate Array (FPGA) device, wherein the FPGA includes one or more configurable digital logic gates that have been configured as a mapping and alignment unit to perform read mapping and alignment.

In some implementations, the computer is configured to access the one or more memory devices using one or more wired or wireless networks, wherein a Field Programmable Gate Array (FPGA) device and the one or more memory devices are housed in an expansion card that has been coupled to a circuit board of a sequencer, wherein the sequencer is configured to generate sequence reads based on an input sample and store the generated sequence reads in the one or more memory devices, and wherein the mapping and alignment unit of the FPGA is configured to access the one or more memory devices to obtain the generated sequence reads.

In some implementations, the computer and the sequencer are each configured to access the one or more memory devices using one or more wired or wireless networks, wherein the Field Programmable Gate Array (FPGA) device and the one or more memory devices are housed in an expansion card that has been coupled to a circuit board of server that is located remotely from the computer and the sequencer, wherein the sequencer is configured to generate sequence reads based on an input sample, provide the generated sequence reads to the server using the one or more wired or wireless networks for storage, of the generated sequence reads, in the one or more memory devices, and wherein the mapping and alignment unit of the FPGA is configured to access the one or more memory devices to obtain the generated sequence reads.

These and other aspects of the present disclosure are discussed in more details in the detailed description below with reference to the accompanying drawings.

BRIEF DESCRIPTION OF THE DRAWINGS

FIG. 1 is a contextual diagram of an example of a system for correlated error mitigation for variant calling.

FIG. 2 is flowchart of an example of a process for correlated error mitigation for variant calling.

FIG. 3 is a flowchart of an example of a process for mapping error mitigation for variant calling.

FIG. 4 is a line chart of an example of a function for translating a an example of an output value from a mapping and alignment unit representing a first mapping confidence score to a second mapping confidence score ($\mu$).

FIG. 5 is a flowchart of an example of a process for sequencing error mitigation for variant calling.

FIG. 6 is another flowchart of an example of a process for correlated error mitigation for variant calling.

FIG. 7 is another flowchart of an example of a process for correlated error mitigation for variant calling.

FIG. 8 is a block diagram of system components that can be used to implement a system for correlated error mitigation for variant calling.

FIG. 9A is an example of summary of experimental results from execution of a process for correlated error mitigation for variant calling that has been performed on a pileup of sequencing reads whose result indicates an example of a true positive results.

FIG. 9B is an example of a modified set of probability results for the pileup of FIG. 9A.

FIG. 10A is an example of summary of experimental results from execution of a process for correlated error mitigation for variant calling that has been performed on a pileup of sequencing reads whose result indicates a low likelihood of an occurrence of a mapping error.

FIG. 10B is an example of a modified set of probability results for the pileup of FIG. 10A.

FIG. 11A is an example of summary of experimental results from execution of a process for correlated error mitigation for variant calling that has been performed on a pileup of sequencing reads whose result indicates a high likelihood that the candidate alt allele is unlikely to be a true variant due to a sequencing error.

FIG. 11B is an example of a modified set of probability results for the pileup of FIG. 11A.

FIG. 12A is an example of summary of experimental results from execution of a process for correlated error mitigation for variant calling that has been performed on a pileup of sequencing reads whose result indicates a high likelihood that the candidate alt allele is unlikely to be a true variant due to a sequencing error on both read orientations.

FIG. 12B is an example of a modified set of probability results for the pileup of FIG. 12.

DETAILED DESCRIPTION

Aspects of the present disclosure are directed towards methods, systems, and apparatus, including computer programs, for accounting for errors that are problematic for variant callers.

Correlated error events cause conventional variant callers to produce genotyping errors, because the internal probability math used by conventional variant callers is typically based on an assumption that errors are uncorrelated. There are two phenomena that tend to produce highly correlated errors in variant calling: (1) mapping errors and (2) sequence-specific errors. Mapping errors occur when a read is mapped to a particular location of a reference genome other than the true origin of the read. Sequence-specific errors, referred to in this specification as sequencing errors or systematic errors, occur because certain sequences of bases tend to produce sequencing errors with high probability. Both types of errors can lead to high-confidence false positives and other genotyping errors in conventional variant callers.

Some variant callers attempt to mitigate these problems with ad hoc rules for filtering reads and variants, but such rules do not approach the performance limits that are possible with more sophisticated algorithms. Other variant callers use machine learning to recognize and suppress such errors, but machine learning has other drawbacks such as being "brittle" by having difficulty in dealing with scenarios that were not represented in the training data or being "opaque," because their outputs cannot be explained. The present disclosure describes new methods for dealing with both types of errors. In particular, the present disclosure addresses the likely fact that certain errors are correlated in a pile up of reads into the probability calculation, rather than relying on a collection of ad hoc rules or machine learning to account for the correlated nature of these types of errors.

For purposes of this disclosure, a "correlated error event" is a category of errors that refers to two or more mapping errors or two or more sequencing errors. The processes described herein can be applied to account for a single type of correlated error event such as one or more mapping errors or one or more sequencing errors. Alternatively, the processes described herein may be applied to account for multiple types of correlated errors such as one or more mapping errors and one or more sequencing errors.

Correlated Error Event Mitigation Systems

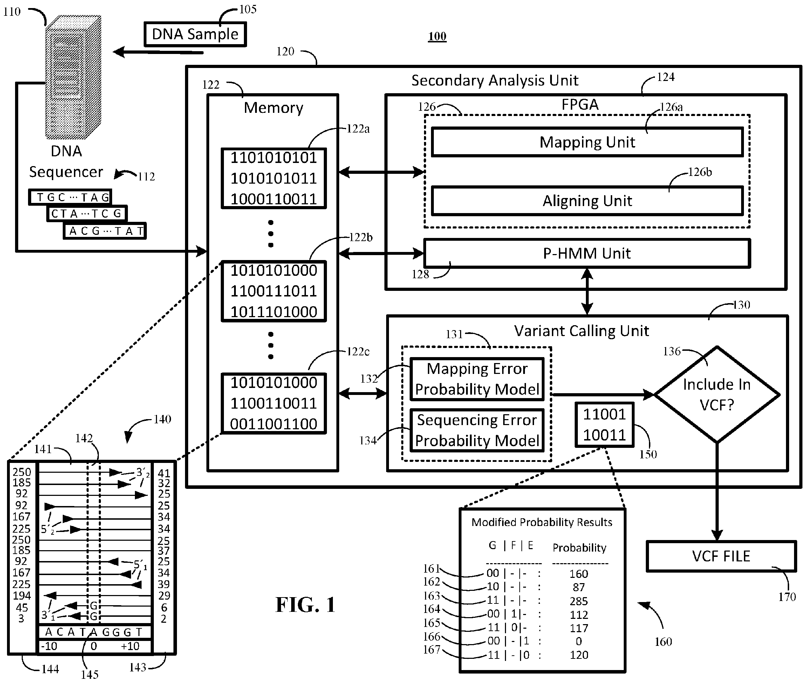

FIG. 1 is a contextual diagram of an example of a system 100 for correlated error event mitigation for variant calling. The system 100 includes a nucleic acid sequencer 110 and a secondary analysis unit 120.

The nucleic acid sequencer 110 is configured to perform primary analysis of a biological sample 105 to translate raw, physical signals detected by the sequencing instrument into an ordered series of nucleotide base calls with associated quality scores. Primary analysis is specific to the nature to the sequencing technology employed. In some implementations, for example, the nucleotides can be detected by sensing changes in fluorescence, electrical charges, electrical currents, or radiated light, or any combination thereof. In some embodiments, the biological sample comprises DNA, RNA, PNA, LNA, chimeric or hybrid forms of nucleic acids.

The nucleic acid sample may be a purified sample or a crude DNA sample containing, for example, lysate derived from a buccal swab, paper, fabric or other substrate that may be impregnated with saliva, blood, or other bodily fluids. In some implementations, the nucleic acid sample may include low amounts of, or fragmented portions of DNA, such as genomic DNA. In some implementations, target sequences can be present in one or more bodily fluids including but not limited to, blood, sputum, plasma, semen, urine and serum. In some implementations, target sequences can include nucleic acids obtained from non-human DNA such a microbial, plant, or entomological DNA.

Primary analysis can include receiving a biological sample 105, and generating output data 112, referred to as one or more base calls, each having a quality score, which are assembled into a plurality of "reads," each representing an ordered set of nucleotides in sequence fragments prepared from the received biological sample 105. In some implementations, the biological sample 105 may include a DNA sample and sequencer 110 may perform primary analysis to output a plurality of reads that include an ordered sequence of nucleotides, or bases, from the DNA sample. In such implementations, the order of sequenced nucleotides includes one or more of guanine (G), cytosine (C), adenine (A), and thymine (T) in any combination. In other implementations, the biological sample 105 can include an RNA sample. In such implementations, the order of sequenced nucleotides include one or more of G, C, A, and uracil (U) in any combination. Accordingly, though the example of FIG. 1 describes a DNA sequencer 110 generating output reads based on an input DNA sample, other implementations may include a sequencer 110 that generates output reads based on an RNA sample. Depending on the sequencing methods used, the reads, which include ordered sequence of contiguous base pairs sequenced from a one or more fragments of the biological sample 105 may vary in length from about 30 base pairs to more than 10,000 base pairs. For example, in some implementations, the read length of sequenced fragments may be between about 150 base pairs to 500 base pairs. about 150 base pairs, about 250 base pairs or about 300 base pairs. The reads may be single reads or pair-end reads from a fragment prepared from the biological sample 105

In some implementations, the nucleic acid sequencer 110 includes a next generation sequencer (NGS) that is configured to generate sequence reads 112 for a given sample 105 in a manner that achieves ultra-high throughput, scalability, and speed through the use of massively parallel sequencing technology. In various examples, the NGS enables rapid sequencing of whole genomes, the ability to zoom in to deeply sequenced target regions, utilize RNA sequencing (RNA-Seq) to discover novel RNA variants and splice sites, or quantify mRNAs for gene expression analysis, analyzing epigenetic factors such as genome-wide DNA methylation and DNA-protein interactions, sequencing of cancer samples to study rare somatic variants and tumor subclones, and studying of microbial diversity in humans or in the environment.

The nucleic acid sequencer 110 is configured to generate the sequence reads 112 and provide the generated sequence reads 112 to a secondary analysis unit 120. The secondary analysis unit 120 may include one or more memory devices 122 and one or more computers such as a Field Programmable Gate Array 124 and a Variant Calling Unit 130. The one or more computers can include one or more devices configured to perform one or more operations. The one or more computers can include only hardware, only software, or any combination thereof.

In some implementations, the secondary analysis unit 120 may be integrated with the nucleic acid sequencer 110. In such implementations, for example, each of the one or more components of the secondary analysis unit 120 can be housed in an expansion card such as a Peripheral Component Interconnect (PCI) expansion card and installed into the nucleic acid sequencer 110. In other implementations, for example, each of the one or more components of the secondary analysis unit 120 can be part of another computer that is different than the nucleic acid sequencer 110 and directly connected to the nucleic acid sequencer 110 using an Ethernet cable, a USB cable, a USB-C cable, or the like. In yet other implementations, for example, each of the components of the secondary analysis unit 120 is integrated into a cloud-based server that is remotely accessible by the nucleic acid sequencer 110 using one or more wired or wireless networks such as local area network (LAN), a wide area network (WAN), a cellular network, the Internet, or a combination thereof. In yet other implementations, for example, one or more of the components of the secondary analysis unit 120 are integrated into the nucleic acid sequencer 110 and one or more of the components of the secondary analysis unit 120 are integrated into another computer such as a cloud-based server. In such implementations, for example, the FPGA 124 that is used to implement the mapping and aligning unit 126 is integrated into the nucleic acid sequencer 110 and the memory 122 and the variant calling unit 130 is integrated into another computer such as a cloud-based server.

Each of these components discussed with reference to FIG. 1, including the nucleic acid sequencer 110, the secondary analysis unit 120, and one or more other computers such as one or more cloud-based servers, if not enabled to communicate with a direct connection, can alternatively, or in addition, be enabled to communicate via one or more wired or wireless networks including one or more of a LAN, a WAN, a cellular network, the Internet, or a combination thereof. Similarly, each of the components of the secondary analysis unit 120 may be configured to communicate with each other, or a component external to the secondary analysis unit 120, using one or more one or more busses, one or more direct connections, or one or more networks to achieve the interaction between respective components of described herein.

The secondary analysis unit 120 is configured to receive the reads 112 and store the reads 112 in a first portion 122a of the memory 122. The field-programmable gate array (FPGA) 124 is dynamically configurable to implement one or more modules of a genomic data analysis pipeline. For example, the FPGA 124 can be dynamically configured to implement a mapping and aligning unit 126, a pair Hidden Markov Model (P-HMM) unit 128, or both. In some implementations, the mapping and aligning unit 126 is a single functional module. In other implementations, the mapping and aligning unit 126 is separated into two discrete functional modules that include a dedicated mapping unit 126a and a dedicated aligning unit 126b. In some implementations, the FPGA 124 is configured to implement, at a particular time, both the mapping and aligning unit 126 and the P-HMM unit 128.

However, in other implementations, the FPGA 124 can be dynamically reconfigured on demand to implement a particular genomic analysis module, or any of the other computers described herein, at any given time. For example, The FPGA 124 can first be configured to include a mapping and aligning unit 126, and then once mapping and aligning operations have been performed by the FPGA 124 on the reads obtained from the memory 122, then later the FPGA 124 can be dynamically reconfigured as a P-HMM unit 128. The FPGA 124 can be dynamically reconfigured from one genomic analysis module to another genomic analysis module, or other computers, on demand, as dictated by any particular genomic analysis workflow. For purposes of the present disclosure, the terms unit and module are used interchangeably to mean one or more hardware components, one or more software components, or any combination thereof, configured to perform one or more specific operations.

With reference to the FPGA unit 124, implementations of the functional operations of the respective mapping and aligning unit 126 and P-HMM unit 128 can be achieved in hardware by programming programmable digital logic gates using a hardware description programming language such as Very High Speed Integrated Circuit (VHSIC) Hardware Description Language (VHDL) to dynamically configure, or reconfigure, the programmable logic gates. Alternatively, though the FPGA 124 can be used to implement the mapping and aligning unit 126 and P-HMM unit 128, the present disclosure need not be so limited. For example, in other implementations, one or more of the mapping and aligning unit 126 and the P-HMM unit 128 may also be implemented on one or more computers local to the DNA sequencer, or remotely from the DNA sequencer, using software. In yet other implementations, the FPGA 124 may also be configured to perform the functions of the variant calling unit 130 to perform an analysis of modified probability results for determination of whether such probability results should be included in a VCF file 170 generated by the variant calling unit 130.

However, the present disclosure is not limited to the use of a dynamically reconfigurable FPGA 124 to implement one or more of the mapping and alignment unit 126, the P-HMM module 128, or other computers, of the secondary analysis unit 120 such as the variant calling unit 130 described here. Instead, other types of programmable or non-programmable integrated circuits can be used. For example, one or more Application-Specific Integrated Circuits (ASICs) can be programmed to perform the functions of one or more of the respective genomic analysis modules, or other computers, described herein. ASICs include integrated circuits that include one or more programmable logic circuits that are similar to the FPGAs described herein in that the digital logic gates of the ASIC are programmable using a hardware description language such VHDL. However, ASICs differ from FPGAs in that ASICs are programmable only once and cannot be dynamically reconfigured once programmed. Furthermore, aspects of the present disclosure are not limited to implementing the genomic analysis modules, or other computers, of the secondary analysis unit 120, using FPGAs or ASICs. Instead, any of the genomic analysis modules, or other computers, of the secondary analysis unit 120 can be implemented using one or more central processing units (CPUs), graphical processing units (GPUs), or any combination therefore that implement the genomic analysis modules, or other computers, of the secondary analysis unit 120 through the execution of software instructions.

In some implementations, the mapping and aligning unit 126 can be implemented using an FPGA 124 that is configured to map and align the generated reads 112 that are stored in a first portion 122a of the memory 122 to a reference genome that is stored in another portion 122b of the memory 122. However, the present disclosure is not limited to storing the reads in the memory 122, accessing the reads from memory 122, storing the reference genome in memory 122, or accessing the reference genome in memory 122. Instead, in some implementations, the generated reads 112, the reference genome, or both, can be stored in a memory device in a cloud based server that is accessible via one or more networks.

Mapped and aligned reads can be output by the mapping and aligning unit 126 for storage in the memory 122 at a third portion 122c of the memory 122. In some implementations, the writes to the memory 122 that include mapped and aligned reads from the FPGA 124 that are referred to as being stored in the third portion 122c may actually be stored in the memory 122 in a manner that overwrites the originally generated reads 112 output by the nucleic acid sequencer 110 and stored in the first portion 122a. Accordingly, though multiple stages of information are shown as being stored in the memory 122 at the first portion 122a, the second portion 122b, and the third portion 122c, respectively, there is no requirement of the present disclosure that all of the data described by this disclosure as being stored in one of these respective portions of memory 122 exist in the memory 122 at any particular time during execution of the processes disclosed by the present disclosure, though there may exist times when all of the data described by this specification is stored in the memory 122 at the same time.

In some implementations, the memory 122 may include a single memory device or multiple memory devices. The use of additional memory devices can reduce lag in accessing data and increase throughput by enabling writing and reading to a fast memory device such as Flash memory as opposed to requests to read or write to one or more disk storage devices accessed using multiple levels of a memory hierarchy used to create the illusion of a fast memory.

Similarly, in some implementations, the use of integrated circuits such as an FPGA 124, ASIC, CPU, GPU, or combination thereof, to implement each genomic analysis module, or other computer, of the secondary analysis unit 120 can include a single FPGA 124, a single ASIC, a single CPU, a single GPU, or any combination thereof. Alternatively, or in addition, the use of integrated circuits such as FPGA 124, ASIC, CPU, GPU, or combination thereof, to implement each genomic analysis module, or other computer, of the secondary analysis unit 120 can include multiple FPGAs 124, multiple ASICs, multiple CPUs, or multiple GPUs, or any combination thereof. The use of additional integrated circuits such as multiple FPGAs to implement a genomic analysis unit, or other computer, of the secondary analysis unit 120 can reduce the amount of time it takes to perform secondary analysis operations such as mapping, aligning, P-HMM probability calculations, and variant calling. In some implementations, use of the FPGA to implement these secondary analysis operations can reduce the time it takes to complete these secondary analysis operations from 24 hours, or more, to as little as 30 minutes, or less. In some implementations, the use of the multiple FPGAs to perform these secondary analysis operations can result in the completion of these secondary analysis operations in as little as 5 minutes.

The output of the mapping and aligning unit 126 includes a pileup of reads that have been mapped and aligned to a reference genome. A pileup may include a text-based format for summarizing base calls of aligned reads, from a DNA sample, to a reference genome or a portion of a reference genome. This output may be stored in one or more portions 122b, 122c of the memory 122 in computer-readable binary format that can be accessed and analyzed by one or more of the FPGA 124, the P-HMM Unit 128, and the variant calling unit 130. Alternatively, the output of the mapping and aligning unit 126 may be stored in a memory, using a computer-readable binary format, of one or more remote computers such as a remote cloud-server accessed using one or more networks. A human-friendly depiction of the output of the mapping and aligning unit 126 of the FPGA 124 can be depicted on a graphical user interface of a user device. An example of such a graphical user interface is shown with reference to interface 140.

The interface 140 can be provided for display on a user interface of a user device that can access the memory 122 of the secondary analysis unit 120. For example, in some implementations, the secondary analysis unit 120 has an attached display device. Alternatively, or in addition, other devices having a display such as a smartphone or tablet can connect to the same network as the secondary analysis unit 120, access the memory 122, and then display pileups such as the pileup 141 of interface 140. In such implementations, the variant calling unit 130 can (i) access the output of the FPGA 124 that mapped and aligned the obtained reads 112 to a reference genome stored in the memory and (ii) generate rendering data, that when rendered on a display device, causes data representing the reads that have been mapped and aligned to the reference genome by the FPGA 124 and stored in the memory 122 to be output for display on a user device in a human-friendly manner that can be read by a person using the interface 140.

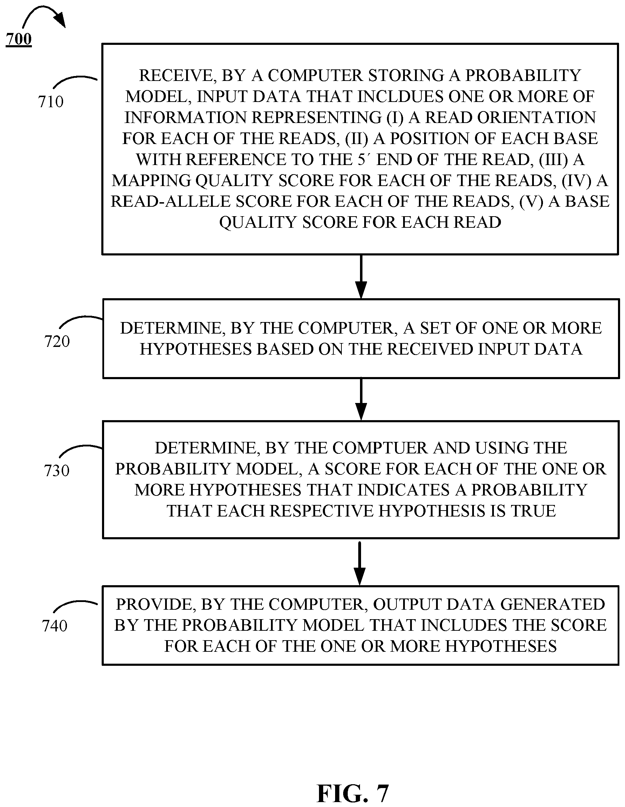

The interface 140 shows that the output of the FPGA 124 includes a pileup 141 of mapped and aligned reads. In this example, the interface 140 represents fourteen reads, respectively, with fourteen respective horizontal lines. These reads are grouped based on the DNA strand from which the reads were generated. For example, the pileup 141 of mapped and aligned reads in the example of FIG. 1 includes a first set of reversed aligned reads that extend in a first direction from the $5'_1$ end of a read leftwards towards the $3'_1$ end of the read and a second set of forward aligned reads that extend in a second direction from the $5'_2$ end of a read rightward towards the $3'_2$ end of the read. Accordingly, in this example of interface 140 of fourteen mapped and aligned reads output from the FPGA 124, the bottom seven reads represent a first set of mapped and aligned reads and the top seven reads represent a second set of mapped and aligned reads. The respective 5' or 3' ends of the two middle reads, which are not shown in interface 140, occur outside of the window 140. Though this example, which is used for illustrating concepts of the present disclosure, depicts a pileup of only fourteen reads, the present disclosure is not so limited. Also, while the pileup 141 displays the reverse aligned reads on the bottom of the pileup and the forward aligned reads on the top of the pileup, other alternatives may exist. For example, as shown with reference to the examples of FIGs. 7-10, the forward aligned reads can be presented on the bottom of the pileup 141 and the reverse aligned reads can be presented on the top of the pileup.

Though an example of an interface 140 is provided that can display information describing characteristics of sequence reads, there is no requirement that any implementations of the present disclosure output information describing characteristics of sequencer reads on a display or that the variant calling unit 130, or other component described herein, would access information from the interface 140. Instead, the interface 140 is merely provided to describe examples of the types of information that constitute characteristics of sequence reads.

The present disclosure may use the FPGA 124 to produce pileups of mapped and aligned reads that includes tens of reads, hundreds of reads, thousands of reads, or more, as necessary for a particular genomic sequence analysis workflow. By way of example, high throughput next generation sequencing of nucleic acid samples can result in hundreds of thousands of short reads that need to be mapped and aligned to one or more regions of a reference genome sequence, or a portion thereof. Mapping and aligning such large read quantities can result in large numbers of overlapping or duplicative short sequence nucleic acid reads. In some implementations, for example, the number of overlapping or duplicative short sequence reads can include 1x, 5x, 10x, 30x, 100x, or more, coverage for one or more respective reference locations of the reference genome sequence. "30x coverage" refers to a mapping and alignment of short reads that includes, for example, a pileup of 30 or more overlapping reads for one or more reference locations of a reference genome sequence. By way of another example, "5x coverage" refers to a mapping and alignment of short reads that includes, for example, a pileup of 5 or more overlapping reads for one or more reference locations of a reference genome sequence.

Methods for Mitigating Correlated Errors

Accordingly, new read processing methods need to be designed to accurately and efficiently map and align a large number of reads to a reference genome sequence, or a portion thereof. For example, data arising from sequencing of a human genome can result in hundreds of millions of short reads that typically need to be mapped and aligned to a position in a complete reference genome before the short reads can be further analyzed for potential variants to determine their biological, diagnostic and/or therapeutic relevance.

The pileup of overlapping reads enables comparisons of each of the different reads at a particular reference location of a reference genome sequence. Analysis of multiple overlapping reads for a particular reference location allows for an accurate determination to be made as to whether there is a true variation, variant, or deviation in the reads mapped and aligned to a particular location of a reference genome or if there may be an error in any one read at the position in question in the pileup. For example, if only one or two of the reads out of the 30 reads that detected a particular nucleotide at position "X" of a reference genome sequence, and each of the 28 or 29 other reads support a determination that another nucleotide exists at the position "X," then the two outlying reads can be excluded as being in error for position "X."

Analysis of the pileup of overlapping reads can enable a more accurate analysis of reads, relative to methods that do not evaluate the similarities or differences of overlapping reads, to determine how a subject's genome differs from a reference genome, e.g., a model genome. For example, analysis of a pileup of overlapping reads can yield more accurate identification of errors, such as chemical errors, machine errors, or read errors, and distinguish such errors from true variants. More specifically, where a subject has a true variant at a position "X" of a reference genome, the majority of reads in the pileup should support that a true variant exists by, e.g., a majority of the reads including the true variant. Statistical models, such as those described herein, can then be implemented to determine a true genetic sequence of a subject with all its true variants from a reference genome.

Accordingly, in various instances, once reads have been generated for a nucleic acid sample, their sequential order aligned, and the generated reads have been mapped to a reference genome, or portion thereof, a true genetic sequence of the subject's genome can be determined. Once the true sample genome is determined, one or more true variations can be determined based on a comparison of the true sample genome to the reference genome, or a portion thereof. Once the one or more variations between the true sample genome and the reference genome, or portion thereof, is determined, a list of all the true variants, or deviations, between the sample genome and the reference genome, can be determined and called out. Such variations can be due to a variety of reasons, and can have biological, diagnostic, and/or therapeutic relevance.

The exemplary pileup depicted by interface 140 shows that the output of the FPGA's 124 mapping and aligning unit 126 includes a base quality score 143 and a mapping confidence score 144 for each of the reads of the pileup. The base quality score 143 includes a value that indicates a level of confidence that the base called for a read at a particular position of interest such as the position "0" 142 is accurate. In the example of FIG. 1, the base quality scores are represented by a range of values defined by a high a base quality score of "41," which indicates a high level of confidence that the base call for a read at position "0" 142 is accurate, and a low base quality score of "2," which indicates a low level of confidence that the base call for a read at position "0" 142 is accurate. In some implementations, the base quality score is an output of the nucleic sequencer 110 that is received by the secondary analysis unit 120 and can be determined using a phred-scale probability of base call error $Q_{phred-base}$, where $Q_{phred-base}$, = -10*log10($P_{e-base}$). In this example, $P_{e-base}$ is the probability of a base calling error for a particular read. In some implementations, a low base quality score may be a factor that is indicative of a sequencing error.

The mapping confidence score 144 indicates a level of confidence that an obtained read 112 was accurately mapped by the mapping and aligning unit 126 to the reference genome 145 at a particular position of interest such as the position "0" (indicated by reference numeral 142). In the example of FIG. 1, the mapping confidence scores are represented by a range of values defined by a high a mapping confidence score of "250," which indicates a high level of confidence that the read was accurately mapped to the reference genome 145 at position "0" 142, and a low mapping confidence score of "0," which indicates a low level of confidence that the read was accurately mapped to the reference genome 145 at position "0" 142. In some implementations, the mapping confidence score is an output of the mapping and aligning unit 126 and can be determined using a phred-scale probability of mapping error $Q_{phred-mapping}$, where $Q_{phred-mapping}$, = -10*log10($P_{e-mapping}$). In this example, $P_{e-mapping}$ is the probability of a mapping error for a particular read. The mapping confidence score 144 value can be proportional to the difference between best alignment score from an aligning algorithm such as a Smith-Waterman aligner and the second best score of the aligner. In some implementations, the adjustments to this method can be made to account for the number of secondary alignments. In some implementations, a low mapping score may be a factor that is indicative of a mapping error.

The interface 140 also shows that the output of the FPGA includes the base nucleotide that was called at the position "0" 142. In the example of FIG. 1, the top twelve reads of the pileup 141 were determined to have the same base call at position "0" 142 as the reference genome. The example interface of 140 depicts this determination by not depicting a letter A, C, G, or T representative of a nucleotide for each of the top twelve reads at position "0" 142. Accordingly, based on a review of the information depicted in interface 140, it can be determined that the top twelve reads have a base call of "A" (adenine) at position "0" 142. Interface 140 also shows that an alt allele of G (guanine) was determined as the base call for the last two reads of the pileup. G (guanine) is an alt allele because it is differs from the nucleotide base of the reference genome at position "0".

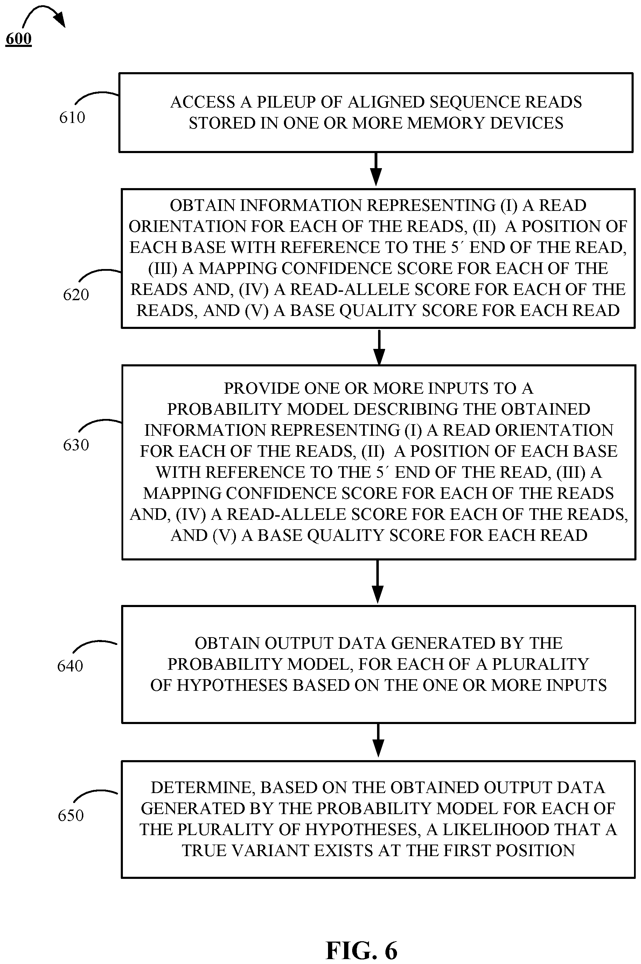

The output of the FPGA also includes additional information that can be determined from an analysis of the pileup 141 shown in the interface 140. First, information describing the strand direction for each read, also referred to as read orientation for each read, can be determined. For example, interface 140 shows that each of the alt alleles occur in the same strand direction or same read orientation. In this example, such information is evidenced by the fact that the alt allele (e.g., "G") occurs in the first set of reads in the reverse aligned direction. By way of another example, the information describing strands may also include the strand direction for each read of the pileup, which may be determined with reference to the respective 3' and 5' ends. Second, information describing the location of the alt alleles within each read can also be determined. For example, a proximity between an alt allele of each read at position "0" 142 from the 5' of their end the read can be determined. In this example, the determined alt alleles "G" occur far from the 5' end of their respective reads, as each alt allele is near the opposite 3' end. The further the alt allele occurs from the 5' end, the more likely the alt allele is associated with a sequencing error. Third, the base quality for each read at position "0" 142 can be determined. By way of example, the base quality for the reads with the alt allele "G" is 6 and 2, respectively. Fourth, the mapping confidence score for each read can be determined for each read at the reference position "0." By way of example, the reads with alt allele "G" at position "0" 142 have a mapping confidence score of 45 and 3, respectively. The information shown in interface 140 for purposes of this example is merely an example to illustrate features of the present disclosure. However, real-world examples of such interfaces are shown with reference to FIGS. 6-9.

Each type of information displayed by interface 140 can be referred to as a characteristic of one or more DNA reads. The characteristics include read-specific characteristics such as base quality scores 143 or mapping confidence scores 144. Additional examples of read-specific characteristics are set forth with reference to interface 140 below.

The information displayed by interface 140 is also stored in the memory 122. By way of example, the interface used to generate interface 140 uses information that describes characteristics of DNA reads to generate interface 140 such as information indicating a pileup 141 of mapped and aligned reads, information indicating the reference genome 145 (or portion thereof), information indicating the base quality score for each read 143, information indicating the mapping confidence score 144 for each read, information indicating a base call for each read at the reference position (e.g., position "0" 142), information indicating whether each read includes an alt allele, information indicating an identification of the reads having an alt allele, information indicating the location of each alt allele with reference to the 5' end of the read that includes the alt allele, information indicating the direction (or orientation) of each read (e.g., forward aligned or reverse aligned), information indicating the direction (or orientation) of each read (e.g., forward aligned or reverse aligned) having an alt allele, and information indicating whether an alt allele was determined to occur in the first set of reads in the forward aligned direction or the second set of reads in the reverse aligned direction. Information describing, indicating, or otherwise representing, each of these characteristics can be stored in the memory 122 at, for example, positions 122b, 122c. By way of example, these characteristics may be stored in the memory 122 in machine-readable binary form.

The variant calling unit 130 can obtain the information describing characteristics of the mapped and aligned reads, and shown in interface 140, from the memory 122. For some of the inputs, the variant calling unit 130 can generate inputs to one or more probability models 131 of the variant calling unit 130 using the information describing characteristics of the mapped and aligned reads from the memory 122. The variant calling unit 130 can provide the generated inputs as inputs to the one or more probability models 131. In some implementations, the variant calling unit 130 can also request that the P-HMM unit 128 generate one or more probabilities for input to the one or more probability models 131 used by the variant calling unit 130. By way of example, the variant calling unit 130 can request that the P-HMM unit 128 determine, for each read, a probability of observing a read $r_i$ given a particular candidate allele $G_{m,\phi}$, at the reference position such as reference position "0" 142. In such implementations, for example, the variant calling unit 130 can use the probability value returned by the P-HMM unit 128 as a read-allele score. The variant calling unit 130 can then provide the information describing characteristics of the mapped and aligned read that include (i) information describing characteristics obtained from the memory 112 and/or a remote memory and (ii) information probabilities calculated by the P-HMM unit 128 that describe one or more characteristics of the pileup described by interface 140, or any combination thereof. In some implementations, the variant calling unit 130 can calculate, or receive the results of calculations from another computer of the secondary analysis unit 120, that represent alternative forms of the read-allele score. These different forms of the read-allele score will be described in more detail below.

Note that the memory 122 stores information describing, supporting, or both, the one or more characteristics of reads described by interface 140. In some instances, the types of information described with reference to interface 140 may need to be derived from the information that is actually stored in the memory 122. For example, in some implementations, the memory 122 may store a location of the candidate alt allele such as "G" in interface 140 and the position of the 5' end of the read that includes the candidate alt allele such as "G" in interface 140. Then, based on that information stored in memory 122, the variant calling unit 130, or other component of the secondary analysis unit 120, can determine the distance of the candidate alt allele "G" from the 5' end of the read. In such instances, there is no need for the memory 122 to store the distance of the candidate alt allele "G" from the 5' end because such information can be derived from the information that is stored in memory 122. Other types of information describing characteristics of the sequence reads can be similarly derived from the information that is actually stored describing the characteristic.

The probability models 131 described herein are used by the variant calling unit 130 as described herein to determine a probability score of various candidate genotypes being true. The improvements presented by the present disclosure improve the accuracy of a variant caller in ways that conventional probability models cannot by enabling the present probability models to account for occurrences of correlated error events such as two or more mapping errors and/or two or more sequencing errors. These new probability models 131 include a mapping error probability model 132 and a sequencing error probability model 134. These probability models provide technical advantages as they are not limited by rule-based decision making nor features of predetermined training data sets.

Mapping Error Probability Models

The mapping error probability model 132 has been designed to account for mapping errors, e.g., those errors that occur when multiple regions of a reference genome such as a first region and a second region that include similar, and in some instances nearly identical, base sequences. In such instances, a mapping and aligning unit may incorrectly map a set of reads to the first region instead of the second region due to the similarities of the base sequences in each respective region. The likelihood of a mapping error can be exacerbated when there is a naturally occurring variant at one or more locations within the first region, which may make the sequence identical to the second region. To account for such errors, the mapping error model receives, as inputs to a probability model, a set of information, obtained by the variant calling unit 130 from the memory 122, which describes characteristics of the mapped and aligned reads, from the P-HMM unit 128, or a combination thereof.

To determine a probability that a mapping error has occurred, the mapping error probability model 132 receives inputs obtained by the variant calling unit 130 that include (i) a read-allele score for each read of the pileup stored in the memory 122 for each candidate allele at the reference position such as reference position "0" 142, and (ii) a mapping quality score for each read of the pileup stored in memory 122. In some implementations, the read-allele score can include

a value $P(r_i|G_{m,\phi})$ that represents probability that the sequencing process would produce read r given a DNA molecule containing allele $G_{m,\phi}$. There are various ways to calculate or estimate this value $P(r_i|G_{m,\phi})$.

In implementations using haplotype-based callers such as Genome Analysis Tool Kit (GATK) or the Dragen® platform, a de Bruijn graph may be used to generate a list of containing haplotype $Hk$, where a haplotype represents a sequence of bases extending beyond the reference position such as reference position "0" 142 in one or both directions. A Hidden Markov Model (HMM) such as a P-HMM unit 128 can then be used to calculate read-haplotype scores $P(r_i|H_k)$, which represent the probabilities that the sequencing process would produce read $r_i$ given a DNA molecule containing haplotype $H_k$.

The HMM calculation can account for possible uncertainty in the alignments, summing the probabilities over multiple possible alignments, rather than assuming that the alignment returned by the mapper/aligner is correct. Then, in some implementations, the read-allele score may then be assigned the best score over the haplotypes that contain the allele:

$$P\left(r_i \mid G_{m,\phi}\right) = \max_{k:H_k \to G_{m,\phi}} P\left(r_i \mid H_k\right) \qquad . \qquad \text{Equation (1)}$$

A detailed description of using a variant calling unit 130 to calculate such probabilities using an HMM unit is set forth in more detail in US Pub. No. 2016/0306922, which is herein incorporated by reference in its entirety.

Variant callers other than the haplotype-based callers may use simpler estimates in order to reduce computational complexity. For example, a variant caller may assume that the alignments from the mapper/aligner are correct and estimate the scores accordingly. To detect SNPs, such a variant caller may estimate the read-allele score for each read of the pileup stored in the memory 122 based on the base call $b_i$ and base quality $q_i$ aligned at a reference position such as the reference position "0" 142, as follows:

$$P\left(r_i \mid G_{m,\phi}\right) = \begin{cases} 1 - 10^{-q_i/10} & \text{if } b_i = G_{m,\phi} \\ \dfrac{10^{-q_i/10}}{3} & \text{if } b_i \neq G_{m,\phi} \end{cases} \qquad . \qquad \text{Equation (2)}$$

For indels, such a variant caller may assign scores based on the length of the indel, whether the indel is an insertion or a deletion, and the surrounding sequence context (e.g. period and length of short tandem repeats).

Regardless of whether the column-wise implementations related to SNP detection or the more general implementation related to SNP and indel detection is used, the output of the mapping error probability model 132 includes one or more probabilities that include a score indicating a likelihood that one or more mapping errors occurred at a reference position such as position "0" 142. In some implementations, the mapping error probability model 132 is configured to output two probability scores for two different hypotheses that include (i) a likelihood that the reads at the reference position 142 indicate the occurrence of a homozygous ref with a foreign allele that matches the alt and (ii) a likelihood that the reads at the reference position 142 indicate the occurrence of a homozygous alt with a foreign allele that matches the reference allele (165).

Sequencing Error Probability Models

The sequencing error probability model 134 is a probability model that accounts for sequencing errors, which can occur because certain combinations of nucleotides can confuse a sequencing algorithm into generating an incorrect sequence. As with the mapping error model 132 described above, in some implementations, there may be variations in the inputs that can be provided to the sequencing error probability model 134 based on the complexity of the variant calling unit used. More complex haplotype variant callers can use a read-allele score that is calculated using equation (1) above, whereas other variant callers than the haplotype variant callers can use a read-allele that is calculated using equation (2). In some implementations, the type of read-allele score used may be determined based on whether the variant calling unit 130 is going to be used to detect SNPs only or SNPs and indels.

Regardless of the type of read-allele score used, the sequencing error probability model 134 receives, as inputs from the variant calling unit 130, a set of information, retrieved by the variant calling unit 130 that describes characteristics of the mapped and aligned reads. To determine a probability that a sequencing error has occurred, the sequencing error probability model 134 receives inputs generated, or otherwise obtained, by the variant calling unit 130 that include (i) a read orientation for each read of a pileup stored in memory 122, (ii) a position of each base at the reference position such as position "0" 142 within each read with reference to the 5' end of the read, (iii) a read-allele score for each read in the pileup stored in memory 122 for each candidate allele at the reference position such as reference position "0" 142, and (iv) a base quality score for each read for the base at the reference position such as position "0".

Other variations of the sequencing error probability model may be configured to receive other inputs. For example, in some implementations, the base quality score for each read for the base at the reference position such as position "0" 142 is not required as an input. An example of a scenario where the base quality score for each read for the base at the reference position "0" 142 is where the read-allele score is determined using equation (2). In such instances the base quality score for each read for the base at the reference position such as reference position "0" 142 may be derived from read-allele score determined using equation (2). However, there may be other implementations where the base quality score for each read for the base at the reference position such as position "0" 142 is not required as a dedicated input because it can instead be derived from another received input.

In yet other implementations, another fourth input may be provided to the sequencing error probability model. For example, the sequencing error probability model may be configured to receive inputs describing a number of homopolymers of the reference genome 145 that precede the reference location such as position "0" 142 in the same direction (or read orientation) of the read that includes a candidate alt allele such as candidate alt allele "G" at position "0" of interface 140 in FIG. 1. Note, for example, that three reference allele "Gs" occur in the reference genome 145 before the reference location 142 that are the same as the candidate alt allele "G." This number of homopolymers can be input into the sequencing error probability model as another input. This input describing the number of homopolymers can be added to improve the accuracy of the model.

The output of the sequencing error probability model 134 includes one or more probabilities that include a score indicating a likelihood that one or more sequencing errors occurred at a reference position such as position "0" 142. In some implementations, the sequencing error probability model 134 is configured to output two probability scores for two different hypotheses that include (i) a likelihood that the reads at the reference position 142 indicate the occurrence of a homozygous reference with a sequencing error that matches the alt allele (166) and (ii) a likelihood that the reads at the reference position 142 indicate the occurrence of a homozygous alt with a sequencing error that matches the reference allele (167).

The variant calling unit 130 generates a set of modified probability results 150, for each of a plurality of hypotheses, using conventional probability models and the one or more. The plurality of hypotheses can be determined based on the inputs provided to the one or more probability models 131, the particular one or more probability models 131 used, or a combination thereof. The generated set of modified probability results 150 may include a probability value for each of a plurality of hypotheses that indicates a likelihood that each respective hypothesis is true.

By way of example, in some implementations, if the variant calling unit 130 only generates, or otherwise provides, inputs for the mapping error model 132, then the modified set of probability results 150 will include conventional probabilities for hypotheses 161, 162, 163 and non-conventional probabilities for hypotheses 164, 165 that respectively account for the likelihood that one or more mapping errors occur at the reference location 142, each of which is described in more detail below. By way of another example, if the variant calling unit 130 only generates, or otherwise provides, inputs for the sequencing error model 134, then the modified set of probability results 150 will include conventional probabilities for hypotheses 161, 162, 163 and non-conventional probabilities for hypotheses 166, 167 that respectively account for the likelihood that one or more sequencing errors occur at the reference location 142, each of which is described in more detail below. However, if the variant calling unit 130 generates, or otherwise provides, inputs for both the mapping error model 132 and the sequencing error model 134, then the modified set of probability results 150 generated by the variant calling unit 130 will include conventional probabilities 161, 162, 163 and non-conventional probabilities 164, 164, 166, 167, each of which is described in more detail below. The set of modified probability results 150 may be generated in a computer-readable binary format and provided. The set of modified probability results 150 improves upon the results of conventional probability calculations typically performed by a variant calling unit 129 in that the modified probability results 150 can also include additional probability scores for one or more additional hypotheses 164, 165, 166, 167 that can be used by the variant calling unit 130 to account for the occurrence of one or more mapping errors, one or more sequencing errors, or a combination of both, using the modified probability calculations.

A human readable version of the set of modified probability results 150 can be shown using a graphical user interface on a display of a user device that has access to the set of probability results 150 generated by the variant calling unit 130. An example of such a graphical user interface is shown in the example of FIG. 1 with reference to the interface 160. In some implementations, the display can include, for example, a display device coupled to the secondary analysis unit 120. Each of the probabilities of the modified set of probabilities 150 is discussed below with reference to the display of the probabilities in the interface 160. However, these probabilities may also be obtained and analyzed in machine-readable format by the variant calling unit 130.

Conventional probabilities calculated by the variant caller 130 may include a set of probabilities determined using conventional probability models. These conventional probability models are configured to determine a probability score for each of three hypotheses that include (i) a likelihood that the reads at the reference position 142 indicate the occurrence of a homozygous reference 161, (ii) a likelihood that the reads at the reference position 142 indicate the occurrence of a heterozygous alt 162, and (iii) a likelihood that the reads at the reference position 142 indicate the occurrence of a homozyogous alt. A homozygous reference occurs when both of the alleles at the reference position 142 are the same. In such instances, no alt allele occurs at the reference position 142. A heterozygous alt occurs when one of the alleles at the reference position 142 is an alt allele and the other allele at the reference position 142 is the reference allele. A homozygous alt occurs when both alleles at the reference position 142 are alt alleles. The conventional probability calculations that produce these three hypotheses assume that all reads in a pileup are mapped correctly and that sequencing errors are uncorrelated across the reads.

However, mapping errors commonly occur, and mapping errors and sequencing errors tend to be highly correlated across reads. To account for the occurrence of these errors, the present disclosure employs a variant calling unit 130 that is configured to use one or more modified probability models 131 to generate a modified set of probability results 150 that includes a probability score for four, or more, additional non-conventional hypotheses. In some implementations, such as for a single alt allele (e.g., a reference allele and first alt allele), the modified set of probability results 150 can include a probability score for the four additional non-conventional hypotheses 164, 165, 166, 167 described herein. However, in other implementations where there is more than a single alt allele (e.g., a reference allele, a first alt allele, and a second alt allele), then the modified set of probability results 150 can include more than the four non-conventional hypotheses described herein. In such a scenario, each respective combination of a first alt allele, second alt allele, and reference allele would have a probability score generated for a set of non-conventional hypotheses that correspond to the four non-conventional hypotheses 164, 165, 166, 167 described herein. The modified set of probability results 150 provides an improvement to the variant calling unit 130, when compared with conventional variant callers that do not avail themselves of the probability scores output by the one or more probability models 131, by allowing the variant calling unit 130 to account for the likelihood that one or more correlated error events occurred at a reference location such as reference location 142 of the pileup 141.

The modified set of probability results 150 includes respective non-conventional probability scores for different hypotheses that account for the potential occurrence of one or more mapping errors. These additional probabilities include (i) a likelihood that the reads at the reference position 142 indicate the occurrence of a homozygous ref with a foreign allele that matches the alt (164) and (ii) a likelihood that the reads at the reference position 142 indicate the occurrence of a homozygous alt with a foreign allele that matches the reference allele (165). A foreign allele may include an allele mapped to a base nucleotide of the reference genome 145 at the reference location 142 that was the result of a mapping error. The foreign allele may be incorrectly mapped to a first region of the reference genome that has a sequence of nucleotide bases that is substantially similar, or identical, to one or more second regions of the reference genome 145.

The modified set of probability results 150 includes respective non-conventional probabilities that account for the potential occurrence of one or more sequencing errors. These additional probabilities include (i) a likelihood that the reads at the reference position 142 indicate the occurrence of a homozygous reference with a sequencing error that matches the alt allele (166) and (ii) a likelihood that the reads at the reference position 142 indicate the occurrence of a homozygous alt with a sequencing error that matches the reference allele (167).

The set of modified probability results 150, collectively, represents a probability that a true variant of a base nucleotide at the reference location 142 exists. In addition, each respective probability of the set of modified probability results 150, which include probabilities 161, 162, 163, 164, 165, 166, 167, provides a particular probability score that the particular genotype represented by each hypothesis exists. Here the particular genotype may include a homozygous reference, a heterozygous alt, a homozygous alt, a homozygous ref with a foreign allele that matches the alt, a homozygous alt with a foreign allele that matches the reference allele, a homozygous reference with a sequencing error that matches the alt allele, or a homozygous alt with a sequencing error that matches the reference allele.

The set of modified probability results 150 can be used by the variant calling unit 130 to determine whether a candidate alt allele one or more sample reads is a true variant of a reference allele at the reference position of interest. With reference to the example of FIG. 1, the variant calling unit 130 can use the set of modified probability results 150 to determine whether the candidate alt allele "G" shown in interface 140 is a true variant of the reference allele "A" at the reference position. For example, the variant calling unit 130 can process the set of modified probability results 150, and determine 136 whether data identifying a true variant at the reference location should included in the Variant Call Format (VCF) file 170 generated by the variant calling unit 130.

The variant calling unit 130 can determine whether the candidate alt allele of one or more reads of a sample, such as data describing the reads with candidate alt allele "G" shown in interface 140, should represents a true variant by determining a collective score based on the modified probability results 150 and evaluating the collective score using one or more predetermined thresholds. The collective score can indicate likelihood that the true variant exists. In some implementations, the collective score can be determined using equation (14). However, other methods of determining the collective score fall within the scope of the present disclosure. If the computer determines 136, that the collective score satisfies a predetermined threshold, then the computer can add information to a VCF file indicating that a true variant exists at the first position. The information added to the VCF file may include, for example, a position of the candidate alt allele, an identifier of the alt allele, a genotype for the alt allele, and data indicating the collective score. Alternatively, if the computer determines 136that the collective score does not satisfy the predetermined threshold, then the computer can discard the output data and determine that the output data indicates the occurrence of a false positive at the reference position.

In the example shown in FIG. 1, the variant calling unit 130 can determine 136 that a collective score based on the probabilities of the set of modified probabilities 150 fails to satisfy a predetermine threshold, and not include information identifying the candidate alt allele "G" in the VCF file 170 as a true variant. This is because the collective score, based on the set of modified probabilities 140, would indicate a high likelihood that one or more sequencing errors exist because the candidate alt alleles "G" only occurring in a single strand in the forward aligned position with low base quality in a location that is far from the 5' end of their respective reads. Thus, the variant calling unit 130 can determine, based on an evaluation of the set of modified probabilities 150, that the candidate alt allele "G" is not a true variant, and instead a false positive, at the reference position 142 and that no true variant exists at that reference location 142.

The probabilities shown in the interface 160 are merely examples and are provided for the purposes of illustrating an example of the present disclosure. The probabilities shown in 160 are not the results of the actual information described in FIG. 1 being put into the actual probability models described by this specification.

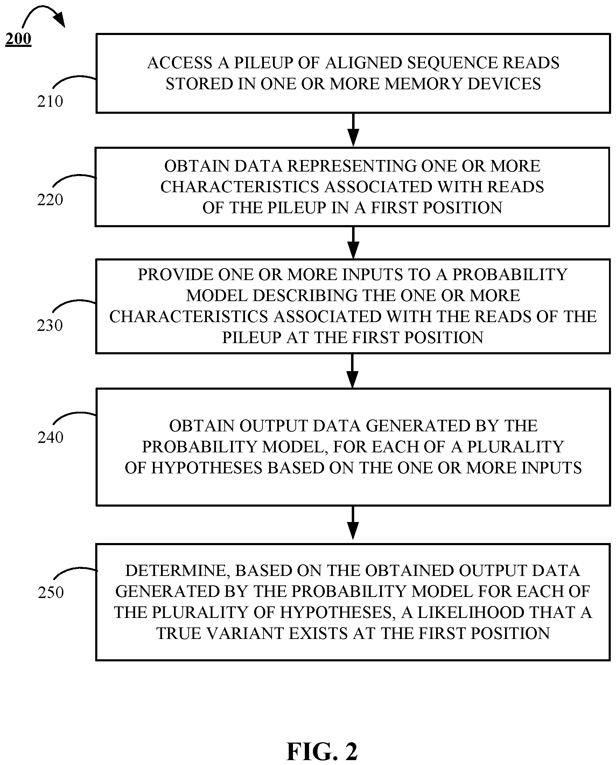

FIG. 2 is flowchart of an example of a process 200 for correlated error event mitigation for variant calling. The process 200 is described below as being performed by a computer such as the variant calling unit of FIG. 1. In some implementations, the variant calling unit may be implemented using hardware such as one or more FPGAs, ASICs, CPUs, GPUs, or any combination thereof, using software, or any combination thereof.

A computer may begin performance of the process 200 by accessing a pileup of aligned sequence reads stored in one or more memory devices (step 210). The aligned sequence reads may have been generated using a mapping and aligning unit implemented using configurable logic gates of an FGPA device. In some implementations, the accessed reads are stored in the one or more memory devices and can include a first set of sequence reads that are forward aligned and a second set of sequence reads that are reverse aligned. Each respective set of sequence read corresponds to a particular read orientation or direction.

The computer can continue performance of the process 200 by obtaining information describing one or more characteristics of respective reads of a pileup at a first position of the pileup of sequence reads (step 220). The one or more characteristics may include attributes of reads in the pileup at the first position that can be used to account for a probability of occurrence of one or more correlated error events.

The computer can continue performance of the process 200 by providing one or more inputs to a probability model describing the one or more characteristics of the reads of the pileup at the first position (step 230). The one or more characteristics associated with the reads of the pileup in the first position may include (i) information describing the one or more characteristics obtained from the one or more memory devices, (ii) information generated by one or more models such as P-HMM model based on the one or more model's processing of information describing the one or more characteristics obtained from the one or more memory devices, or a combination thereof. In some implementations, the probability model is configured to determine, for each hypothesis of one or more hypotheses selected based on the one or more inputs, a score for each of the hypothesis that indicates that the hypothesis is true.

The computer can continue performance of the process 200 by obtaining output information, for each hypothesis of the one or more hypotheses based on the one or more inputs. The output information for each hypothesis can be (i) generated by the probability model based on the probability model's processing of the one or more inputs to the probability model describing the one or more characteristics of the respective reads of the pileup and (ii) indicative of a probability that the hypothesis is true (step 240). In some implementations, the computer can obtain such output information for each of the one or more hypotheses or a subset of the one or more hypotheses. Whether a particular hypothesis will be included in the output information can be determined based on the inputs provided to the probability model.

In some implementations, the one or more hypotheses may include (i) a likelihood that the reads at the reference position indicate the occurrence of a homozygous reference, (ii) a likelihood that the reads at the reference position indicate the occurrence of a heterozygous alt, (iii) a likelihood that the reads at the reference position indicate the occurrence of a homozyogous alt, (iv) a likelihood that the reads at the reference position indicate the occurrence of a homozygous ref with a foreign allele that matches the alt, (v) a likelihood that the reads at the reference position indicate the occurrence of a homozygous alt with a foreign allele that matches the reference allele, (vi) a likelihood that the reads at the reference position indicate the occurrence of a homozygous reference with a sequencing error that matches the alt allele, a (vii) a likelihood that the reads at the reference position indicate the occurrence of a homozygous alt with a sequencing error that matches the reference allele, or any combination thereof, based on the on the one or more inputs to the probability model, as described above.

The computer can continue performance of the process 200 by determining, based on the obtained output data generated by the probability model for each of the plurality of hypotheses, a likelihood that a true variant exists at the first position (step 250). In some implementations, this determination may be made, for example, based on an individual, or collective, evaluation of the probability scores determined for each of the one or more hypotheses against one or more predetermined thresholds.

For example, the computer can determine a collective score that is based on the output data generated by the probability model for each of the plurality of hypotheses. The collective score can indicate likelihood that the true variant exists. In some implementations, the collective score can be determined using equation (14). However, other methods of determining the collective score fall within the scope of the present disclosure. If the computer determines, that the collective score satisfies a predetermined threshold, then the computer can add information to a VCF file indicating that a true variant exists at the first position. Alternatively, if the computer determines that the collective score does not satisfy the predetermined threshold, then the computer can discard the output data and determine that the output data indicates the occurrence of a false positive at the reference position.

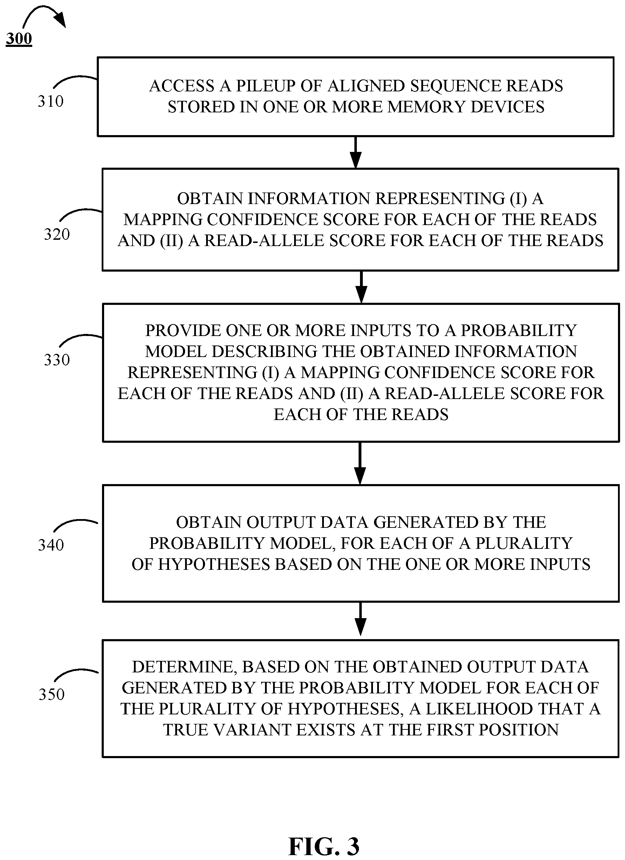

FIG. 3 is a flowchart of an example of a process 300 for mapping error mitigation for variant calling. The process 300 is described below as being performed by a computer such as the variant calling unit of FIG. 1. In some implementations, the variant calling unit may be implemented using hardware such as one or more FPGAs, ASICs, CPUs, GPUs, or any combination thereof, using software, or any combination thereof.

A computer can begin performance of the process 300 by accessing a pileup of aligned sequence reads stored in one or more mem ory devices (step 310). The aligned sequence reads may have been generated using a mapping and aligning unit implemented using configurable logic gates of an FGPA device. In some implementations, the accessed reads are stored in the one or more memory devices and can include a first set of sequence reads that are forward aligned and a second set of sequence reads that are reverse aligned. Each respective set of sequence read corresponds to a particular read orientation or direction.

The computer can continue performance of the process 300 by obtaining information describing (i) a mapping confidence score for each of the reads of the pileup of aligned sequence reads stored in the one or more memories and (ii) a read-allele score for each of the reads of the pileup of aligned sequence reads stored in the one or more memories (320) for each candidate allele at the reference position.

In some implementations, the mapping confidence score can include an output of the mapping and aligning unit 126 and can be determined using a phred-scale probability of mapping error $Q_{phred-mapping}$, where $Q_{phred-mapping}$, = $-10*log10(P_{e-mapping})$. In this example, $P_{e-mapping}$ is the probability of a mapping error for a particular read. The mapping confidence score 144 value can be proportional to the difference between best alignment score from an aligning algorithm such as a Smith-Waterman aligner and the second best score of the aligner.

The read-allele score may be determined in a number of different ways. For example, a more complex haplotype variant caller can use a read-allele score that is calculated using equation (1) above. Alternatively, other variant callers than the haplotype variant callers can use a read-allele score calculated using equation (2) above. In some implementations, the type of read-allele score used may be determined based on whether the variant calling unit 130 is going to be used to detect SNPs only or SNPs and indels. For example, in some implementations where a variant calling unit will be used to detect SNPs and indels, then the read-allele score calculated using equation (1) above can be used. By way of another example, in other implementations where a variant calling unit will be used to detect only SNPs, then the readl-allele score calculated using equation (2) can be used.

The computer can continue performance of the process 300 by providing one or more inputs to a probability model describing the obtained information describing (i) the mapping confidence score for each of the reads of the pileup of aligned sequence reads stored in the one or more memories and (ii) the read-allele score for each of the reads of the pileup of aligned sequence reads stored in the one or more memories (step 330) for each candidate allele at the reference position.

The computer can continue performance of the process 300 by obtaining output information, for each hypothesis of the one or more hypotheses based on the one or more inputs obtained at 320 (340). In the example of the process 300, the obtained input includes inputs for a mapping error probability model that include (i) a mapping confidence score for each of the reads and (ii) a read-allele score for each of the reads for each candidate allele at the reference position. Accordingly, based on the receipt of these inputs for the mapping error probability model, the computer will generate output information for one or more hypotheses that account for the occurrence of one or more mapping errors. Such hypotheses include (i) a likelihood that the reads at the reference position indicate the occurrence of a homozygous ref with a foreign allele that matches the alt and (ii) a likelihood that the reads at the reference position indicate the occurrence of a homozygous alt with a foreign allele that matches the reference allele.

The output information includes, for each of these hypotheses, information that is (i) generated by the mapping error probability model based on the probability model's processing of the inputs to the probability model that describe (i) a mapping confidence score for each of the reads of the pileup of aligned sequence reads stored in the one or more memories and (ii) a read-allele score for each of the reads of the pileup of aligned sequence reads stored in the one or more memories for each candidate allele at the reference position. In addition, obtained output information includes a score, for each of the particular hypothesis that account for the occurrence of the one or more mapping errors, that is indicative of a likelihood that the hypothesis is true.

The computer can continue performance of the process 300 by determining, based on the obtained output data generated by the probability model for each of the plurality of hypotheses, a likelihood that a true variant exists at the first position (step 350). In some implementations, this determination may be made, for example, based on an individual, or collective, evaluation of the probability scores determined for each of the one or more hypotheses against one or more predetermined thresholds.

For example, the computer can determine a collective score that is based on the output data generated by the probability model for each of the plurality of hypotheses. The collective score can indicate likelihood that the true variant exists. In some implementations, the collective score can be determined using equation (14). However, other methods of determining the collective score fall within the scope of the present disclosure. If the computer determines, that the collective score satisfies a predetermined threshold, then the computer can add information to a VCF file indicating that a true variant exists at the first position. Alternatively, if the computer determines that the collective score does not satisfy the predetermined threshold, then the computer can discard the output data and determine that the output data indicates the occurrence of a false positive at the reference position.

The process 300 above describes a method for using a probability model that can be used to account for a likelihood of a mapping error. An example of a probability model that can be used to account for a likelihood of one or more mapping errors is described in more detail below.

In one implementation, a probability model is modified to incorporate the possibility that the pileup of sequence reads stored in the memory device includes multiple mismapped reads resulting in the occurrence of one or more mapping errors. The probability model is applicable to the following scenario where: (1) each read $r_i$ is accompanied by a mapping quality $\mu_i$ indicating the phred-scale confidence that it was correctly mapped. Thus $R = \{r_i, \mu_i : i = 1 \ldots N_R\}$; (2) the secondary alignments (i.e., the other loci in the genome where a read aligns well) may be unknown and/or too numerous to tabulate; and (3) as inputs we are given a base quality $P(r_i|G_{m,\phi})$ for each read $r_i$ and candidate allele $G_{m,\phi}$.

In one implementation, the present disclosure modifies conventional variant calling probability models supplementing the list of candidate genotypes with extended candidate genotypes, which represent the hypotheses that the pileup of sequence reads stored in the memory device contains a mixture of local alleles and foreign alleles.

For a diploid genome with one foreign allele, the approach of the present disclosure defines the extended candidate genotype $\mathbf{G'}_m = [G_{m,1} \; G_{m,2} \; F_m]$, where $F_m$ is the foreign allele. The local alleles $G_{m,1}$ and $G_{m,2}$ are each assumed to have allele frequency $(1 - \beta)/2$ while the foreign allele $F_m$ has allele frequency $\beta$, where $\beta$ is unknown.

For each extended candidate genotype $\mathbf{G}_m$, the model calculates:

$$\Psi_m = P(\mathbf{G}_m) \max_{\beta, p_F} \prod_i \left( \underbrace{\beta P(r_i|F_m) U\left(-10\log_{10}\left(\frac{p_F}{P_0(F_m)}\right) - \mu_i\right)}_{\text{term \#1}} + (1-\beta) \underbrace{\frac{P(r_i|G_{m,1}) + P(r_i|G_{m,2})}{2}}_{\text{term \#2}} \right), \qquad \text{Equation (3)}$$

where U(t) is the Heavyside Unit Step function

$$U(t) = \begin{cases} 1 & t >= 0 \\ 0 & t < 0 \end{cases} . \qquad \text{Equation (4)}$$

and $P_0(F_m)$ is the prior probability of the genotype $[\rho \; F_m]$ occurring at the locus of interest, where $\rho$ is the reference allele at the locus of interest. The value $\Psi_m$ is an estimate of the joint probability $P(\mathbf{G'}_m, R)$.

The probability model for determining a likelihood of occurrence of one or more mapping errors is based on a relationship between the mapping quality $\mu_i$ of a mismapped read and the prior probability of the variant (or cluster of variants) that caused read i to be mismapped. In the probability model for determining a likelihood of occurrence of one or more mapping errors, the quantity $p_F$ represents the prior probability that a sufficient number of variants occurred at another location to cause reads to be mismapped with mapping quality $\mu = -10\log_{10}(p_F/P_0(F_m))$. Term #1 above is non-zero only if the mapping quality indicator $\mu_i$ does not exceed this threshold, which increases as $p_F$ decreases. It is generally unknown how many variants may have occurred at a remote location, $p_F$ is swept to find the value that maximizes $\Psi_m$, thereby testing every hypothesis about the number of remote variants that may have caused foreign reads to end up here.

In some implementations, the complexity of the probability model for determining a likelihood of occurrence of one or more mapping errors be optimized. It is noted that evaluating $\Psi_m$ may appear to have high computational complexity, because as shown in (Equation #3) both $\beta$ and $p_F$ are swept independently and the result is evaluated over a continuous range of values. Depending on the resolution, this could have very high computational complexity. However, $p_F$ need only be swept over a discrete set of values corresponding to values of $\mu_i$ where:

$$P(r_i|F_m) > \left(P(r_i|G_{m,1}) + P(r_i|G_{m,2})\right)/2, \qquad \text{Equation (5)}$$

which is typically a small number. In practice, the term $\beta$ does not need to be swept at all; instead the optimal value can be estimated as the fraction of reads where term #1 exceeds term #2, and this estimate usually has negligible impact on the result. In the end, the computational cost of this operation may be typically insignificant relative to the other parts of the system (e.g. the Hidden Markov Model (HMM) calculations).

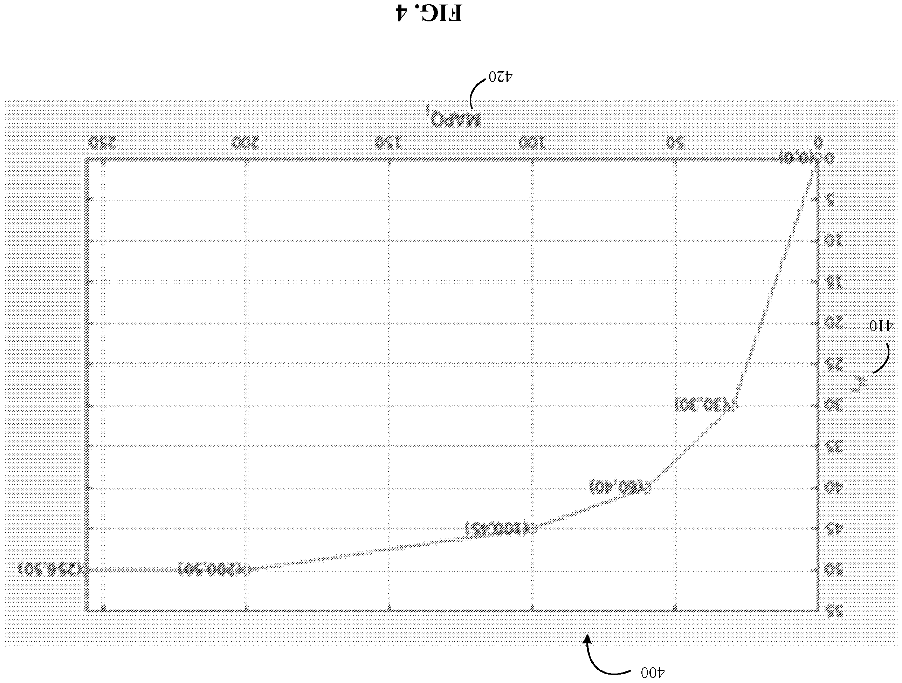

In some implementations, the mapping confidence score may need to be adjusted. Note that, in some implementations, the mapping confidence score reported by the mapping and aligning unit may represent an estimate of the phred-scale confidence that a read is correctly mapped, but in practice, this estimate may be inaccurate. As such, depending on the mapper, it may be beneficial to adjust the mapping confidence score to better match a true likelihood of mismapping. In some implementations, the an output value of the mapping and alignment unit representative of a first mapping confidence score such as a MAPQ score can be translated to mapping confidence score $\mu_i$ using the function 400 shown in FIG. 4.

In some implementations, the term $\beta$ may be limited to the range [0, 0.5]. The value $\beta = 0.5$ corresponds to the scenario where all reads for an alternate reference location get mapped to the reference location of interest. Although it is conceivable that there may be more than one source of foreign reads, which would imply higher possible values of $\beta$, limiting $\beta$ to 0.5 can improve the overall accuracy, recovering some true positives that would otherwise be suppressed.

In some implementations, the number of candidates can be reduced to only cases where $G_{m,1} = G_{m,2} = G_m$. In this case equation (3) simplifies to:

$$\Psi_m = P(\mathbf{G}_m)\max_{\beta,p_F}\prod_i\left(\underbrace{\beta P(r_i \mid F_m)U\left(-10\log_{10}\left(\frac{p_F}{P_0(F_m)}\right)-\mu_i\right)}_{\text{term \#1}}+(1-\beta)\underbrace{P(r_i \mid G_m)}_{\text{term \#2}}\right) \qquad \text{Equation (6)}$$

In some implementations, the expressions above may assume an input that only include reads that overlap the genotyping event, i.e., those that favor one allele over another. However, in some implementations, there may be ambiguity about which reads overlap the event. In such scenarios, the following expression avoids complications associated with including non-overlapping reads in the calculation:

$$\Psi_m = P(\mathbf{G}_m)\max_{\beta,p_F}\prod_i\left(\beta P(r_i \mid F_m)+(1-\beta)P(r_i \mid G_m)-U\left(\mu_i+10\log_{10}\left(\frac{p_F}{P_0(F_m)}\right)\right)\beta\min\left(P(r_i \mid F_m)-P(r_i \mid G_m),0\right)\right) \qquad \text{Equation (7)}$$

In some implementations, another variation on the probability model for determining a likelihood that an occurrence of one or more mapping errors exists. In such implementations, data describing knowledge of the strand direction of each read may be considered by the probability model. Generally, mismapped reads are often confined to a single strand direction, and this can be useful information that supports a hypothesis that such reads are foreign. To account for this potential error, indicators in the probability model for determining a likelihood that an occurrence of one or more mapping errors exits, let $\theta_i$ indicate the strand direction for read $i$, with values 0 and 1 indicating the forward and reverse strand directions, respectively. With a strand-aware modified probability mapping model for determining a likelihood that an occurrence of one or more mapping errors exist, three hypotheses can be evaluated. These three hypothesis include that the foreign reads occur exclusively on the forward strand, exclusively on the reverse strand, or on both strands. A solution includes a hypothesis that maximizes $\Psi_m$. Starting with equation **Error! Reference source not found.,** this variation is as follows:

$$\Psi_m = P(\mathbf{G}_m)\max_{n,\beta,p_F}\left(\prod_{i\notin\Lambda_n}P(r_i \mid G_m)\right)\prod_{i\in\Lambda_n}\left(\beta P(r_i \mid F_m)U\left(-10\log_{10}\left(\frac{p_F}{P_0(F_m)}\right)-\mu_i\right)+(1-\beta)P(r_i \mid G_m)\right), \qquad \text{Equation (8)}$$

where $\Lambda_0 = \{i : \theta_i = 0\}$, $\Lambda_1 = \{i: \theta_i = 1\}$, $\Lambda_2 = \{i : \theta_i = 0 \text{ or } 1\}$.

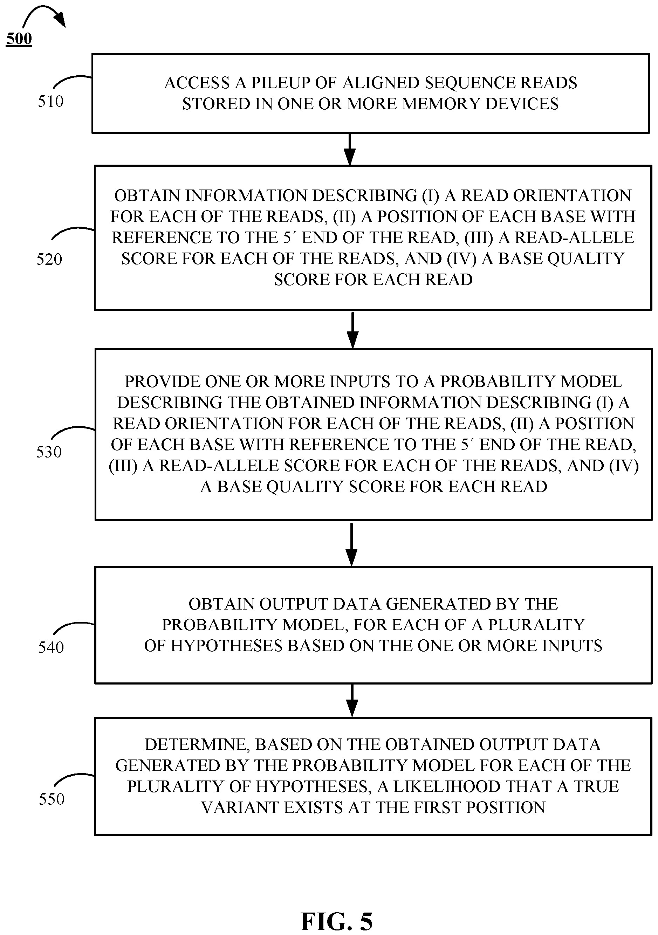

FIG. 5 is a flowchart of an example of a process for sequencing error mitigation for variant calling. The process 500 is described below as being performed by a computer such as the variant calling unit of FIG. 1. In some implementations, the variant calling unit may be implemented using hardware such as one or more FPGAs, ASICs, CPUs, GPUs, or any combination thereof, using software, or any combination thereof.

A computer may begin performance of the process 500 by accessing a pileup of aligned sequence reads stored in one or more memory devices (step 510). The aligned sequence reads may have been generated using a mapping and aligning unit implemented using configurable logic gates of an FGPA device. In some implementations, the accessed reads are stored in the one or more memory devices and can include a first set of sequence reads that are forward aligned and a second set of sequence reads that are reverse aligned. Each respective set of sequence read corresponds to a particular read orientation or direction.

The computer may continue performance of the process 500 by obtaining information describing (i) a read orientation for each of the reads of the pileup of aligned sequence reads stored in the one or more memories, (ii) a position of each base at the reference location with reference to the 5' end of the read for each of the reads of the pileup of aligned sequence reads stored in the one or more memories, (iii) a read-allele score for each of the reads of the pileup of aligned sequence reads stored in the one or more memories (520) for each candidate allele at the reference position, and (iv) a base quality score for each read for the base at the reference position such as position "0".

The read-allele score may be determined in a number of different ways. For example, a more complex haplotype variant caller can use a read-allele score that is calculated using equation (1) above. Alternatively, other variant callers than the haplotype variant callers can use a read-allele score calculated using equation (2) above. In some implementations, the type of read-allele score used may be determined based on whether the variant calling unit 130 is going to be used to detect SNPs only or SNPs and indels. For example, in some implementations where a variant calling unit will be used to detect SNPs and indels, then the read-allele score calculated using equation (1) above can be used. By way of another example, in other implementations where a variant calling unit will be used to detect only SNPs, then the readl-allele score calculated using equation (2) can be used.

The computer can continue performance of the process 500 by providing one or more inputs to a probability model describing the obtained information describing (i) a read orientation for each of the reads of the pileup of aligned sequence reads stored in the one or more memories, (ii) a position of each base at the reference location with reference to the 5' end of the read for each of the reads of the pileup of aligned sequence reads stored in the one or more memories, (iii) a read-allele score for each of the reads of the pileup of aligned sequence reads stored in the one or more memories (step 530) for each candidate allele at the reference position, and (iv) a base quality score for each read for the base at the reference position such as position "0".

The computer can continue performance of the process 500 by obtaining output information, for each hypothesis of the one or more hypotheses based on the one or more inputs obtained at 520 (540). In the example of the process 500, the obtained input includes inputs for a sequencing error probability model that includes (i) a read orientation for each of the reads of the pileup of aligned sequence reads stored in the one or more memories, (ii) a position of each base at the reference location with reference to the 5' end of the read for each of the reads of the pileup of aligned sequence reads stored in the one or more memories, (iii) a read-allele score for each of the reads of the pileup of aligned sequence reads stored in the one or more memories for each candidate allele at the reference position, and (iv) a base quality score for each read for the base at the reference position such as position "0". Accordingly, based on the receipt of these inputs for the sequencing error probability model, the computer will generate output information for one or more hypotheses that account for the occurrence of one or more sequencing errors. Such hypotheses include (i) a likelihood that the reads at the reference position indicate the occurrence of a homozygous reference with a sequencing error that matches the alt allele and (ii) a likelihood that the reads at the reference position indicate the occurrence of a homozygous alt with a sequencing error that matches the reference allele.

The obtained output information includes, for each of these hypotheses, information that is (i) generated by the sequencing error probability model based on the probability model's processing of the inputs to the probability model that describe (i) a read orientation for each of the reads of the pileup of aligned sequence reads stored in the one or more memories, (ii) a position of each base at the reference location with reference to the 5' end of the read for each of the reads of the pileup of aligned sequence reads stored in the one or more memories, (iii) a read-allele score for each of the reads of the pileup of aligned sequence reads stored in the one or more memories for each candidate allele at the reference position, and (iv) a base quality score for each read for the base at the reference position such as position "0". In addition, obtained output information includes a score, for each of the particular hypothesis that account for the occurrence of the one or more sequencing errors, that is indicative of a likelihood that the hypothesis is true.

The computer can continue performance of the process 500 by determining, based on the obtained output data generated by the probability model for each of the plurality of hypotheses, a likelihood that a true variant exists at the first position (step 550). In some implementations, this determination may be made, for example, based on an individual, or collective, evaluation of the probability scores determined for each of the one or more hypotheses against one or more predetermined thresholds.

For example, the computer can determine a collective score that is based on the output data generated by the probability model for each of the plurality of hypotheses. The collective score can indicate likelihood that the true variant exists. In some implementations, the collective score can be determined using equation (14). However, other methods of determining the collective score fall within the scope of the present disclosure. If the computer determines, that the collective score satisfies a predetermined threshold, then the computer can add information to a VCF file indicating that a true variant exists at the first position. Alternatively, if the computer determines that the collective score does not satisfy the predetermined threshold, then the computer can discard the output data and determine that the output data indicates the occurrence of a false positive at the reference position.

The process 500 above describes a method for using a probability model that can be used to account for a likelihood of a sequencing error. An example of a probability model that can be used to account for a likelihood of one or more sequencing errors, also referred to as systematic errors, is described in more detail below.

In one implementation, probability model is modified to account for the observation that certain sequences of bases tend to produce base-call errors with high probability and that this probability is not represented by the base quality used to calculate $P(r_i|G_{m,\phi})$.

The probability model for determining a likelihood of occurrence of a sequencing error is applicable to the following scenario where:

(1) the errors seem to occur independently per strand direction, because it is common for one strand direction to be corrupted while the other strand is free of errors;

(2) the errors are more likely to occur further from the 5' end of the read, and the rate of errors often decreases abruptly at a certain distance from the 5' end. Thus, when reads of a given strand direction are listed in order of decreasing distance from the 5' end, all of the errors are contained within a subset at the start of the list;

(3) the errors are often accompanied by a drop-off in mean base quality over the subset of reads containing the error, but not every erroneous read has low base quality, and the mean base quality is often not low enough to reflect the true error rate associated with these error events; and

(4) the error is often preceded by a homopolymer that matches the error, e.g., a T==>G error is often preceded by a sequence of G's.

Accordingly, the present disclosure provides a probability model for determining a likelihood of occurrence of a sequencing error that accounts for the four characteristics described above.

In one implementation, the probability model for determining a likelihood of occurrence of a sequencing error that accounts for the four characteristics described above can be achieved by beginning with the following term definitions: Let $\theta$ indicate the strand direction, $\theta = 0,1$.

Let the reads $r_{\theta,i}$ be ordered by strand direction and distance from the locus of interest to the 5' end, where i =1 is furthest from the 5' end, which is most likely to be affected by an error event.

Let $q_{\theta,i}$ indicate the phred-scale base quality of the base aligned with the locus of interest for read $r_{\theta,i}$.

Let $\bar{q}_{\theta,n_\theta}$ be the mean base quality over a subset of ordered reads $i = 1...n_\theta$ on strand direction $\theta$ :

$$\bar{q}_{\theta,n_\theta} = \frac{1}{n_\theta}\sum_{i=1}^{n_\theta} q_{\theta,i} \text{ for } n_\theta \geq 1.$$

Let us define the extended candidate genotype $\mathbf{G'}_m = [G_{m,1}\ G_{m,2}\ E_{m,0}\ E_{m,1}]$, where $E_{m,\theta}$ is the error allele for strand direction $\theta$.

Let $L_{E,\theta}$ be the length of the homopolymer matching base $E_{m,\theta}$ immediately preceding the error in strand direction $\theta$.

For each extended candidate genotype $\mathbf{G'}_m$ , we calculate:

$$\Psi_m = P(\mathbf{G}_m)\prod_{\theta=0,1}\max_{r_\theta,\alpha_\theta} f\left(\bar{q}_{\theta,n_\theta}, L_{E,\theta}\right)\left(\prod_{i>n_\theta}\frac{P\left(r_{\theta,i}|G_{m,1}\right)+P\left(r_{\theta,i}|G_{m,2}\right)}{2}\right)\prod_{i\leq n_\theta}\left(\alpha_\theta P\left(r_{\theta,i}|E_{m,\theta}\right)+(1-\alpha_\theta)\frac{P\left(r_{\theta,i}|G_{m,1}\right)+P\left(r_{\theta,i}|G_{m,2}\right)}{2}\right), \quad \text{Equation (9)}$$

where $f(\bar{q}, L_E)$ indicates the prior probability of an error event affecting a subset of reads as a function of the mean base quality over the subset and the length of the homopolymer matching the error.

The quantity $\Psi_m$ represents an estimate of the joint probability $P(\mathbf{G'}_m, R)$ under the following assumptions: (1) the error events affect a contiguous subset of reads when ordered by decreasing distance from the 5' end, starting with the first, and do not affect the reads outside of that subset, (2) the error events happen independently for each strand direction, and (3) the prior probability of such error events is a function of the mean base quality over the subset of reads affected by the error event and the length of the homopolymer matching base $E_{m,\theta}$ immediately preceding the error in strand direction $\theta$.

In some implementations, the number of candidates can be reduced to only test evaluating cases where $G_{m,1} =$

$G_{m,2} = G_m$. In this case the expression simplifies to

$$\Psi_m = P(\mathbf{G}_m) \prod_{\theta=0,1} \max_{n_\theta, \alpha_\theta} f\left(\overline{q}_{\theta,n_\theta}, L_{E,\theta}\right) \left(\prod_{i>n_\theta} P\left(r_{\theta,i} \mid G_m\right)\right) \prod_{i \leq n_\theta} \left(\alpha_\theta P\left(r_{\theta,i} \mid E_{m,\theta}\right) + (1-\alpha_\theta) P\left(r_{\theta,i} \mid G_m\right)\right). \quad \text{Equation (10)}$$

In some implementations, the value of $\alpha_\theta$ can be fixed as $\alpha_\theta = 0.5$. Thus, we can rewrite **Error! Reference source not found.** as:

$$\Psi_m = P(\mathbf{G}_m) \prod_{\theta=0,1} \max_{n_\theta} f\left(\overline{q}_{\theta,n_\theta}, L_{E,\theta}\right) \left(\prod_{i>n_\theta} P\left(r_{\theta,i} \mid G_m\right)\right) \prod_{i \leq n_\theta} \left(\frac{P\left(r_{\theta,i} \mid E_{m,\theta}\right) + P\left(r_{\theta,i} \mid G_m\right)}{2}\right). \quad \text{Equation (11)}$$

In some implementations, the prior probability function $f(\overline{q}_{\theta,n_\theta}, L_{E,\theta})$ in **Error! Reference source not found.** is expressed as a general function that could have a wide range of shapes. In theory, this function could be trained on real data. However, given limitations on the use of training this function on real data, some implementations of the present probability model for determining sequencing errors may use $f(\overline{q}, L_E) = 10^{-\max(0, 2.5\overline{q} - \min(20, 5L_E))/10}$.

In some implementations, the equations above may accommodate the possibility of different error alleles for each strand direction. However, in some implementations, the probability model for determining a likelihood of one or more sequencing errors may only consider hypotheses where $E_{m,0} = E_{m,1}$. In such implementations, the $\theta$ subscript can be dropped and then the error allele can be denoted as $E_m$.

FIG. 6 is another flowchart of an example of a process 600 for correlated error mitigation for variant calling. The process 600 is described below as being performed by a computer such as the variant calling unit of FIG. 1. In some implementations, the variant calling unit may be implemented using hardware such as one or more FPGAs, ASICs, CPUs, GPUs, or any combination thereof, using software, or any combination thereof.

A computer may begin performance of the process 600 by accessing a pileup of aligned sequence reads stored in one or more memory devices (step 610). The aligned sequence reads may have been generated using a mapping and aligning unit implemented using configurable logic gates of an FGPA device. In some implementations, the accessed reads are stored in the one or more memory devices and can include a first set of sequence reads that are forward aligned and a second set of sequence reads that are reverse aligned. Each respective set of sequence read corresponds to a particular read orientation or direction.

The computer may continue performance of the process 600 by obtaining information describing (i) a read orientation for each of the reads of the pileup of aligned sequence reads stored in the one or more memories, (ii) a position of each base at the reference location with reference to the 5' end of the read for each of the reads of the pileup of aligned sequence reads stored in the one or more memories, (iii) a mapping confidence score for each of the reads, (iv) a read-allele score for each of the reads of the pileup of aligned sequence reads stored in the one or more memories for each candidate allele at the reference position, and (v) a base quality score for each read for the base at the reference position such as position "0" (620).

In some implementations, the mapping confidence score can include an output of the mapping and aligning unit 126 and can be determined using a phred-scale probability of mapping error $Q_{phred-mapping}$, where $Q_{phred-mapping,} = -10 * \log 10(P_{e-mapping})$. In this example, $P_{e-mapping}$ is the probability of a mapping error for a particular read. The mapping confidence score 144 value can be proportional to the difference between best alignment score from an aligning algorithm such as a Smith-Waterman aligner and the second best score of the aligner.

The read-allele score may be determined in a number of different ways. For example, a more complex haplotype variant caller can use a read-allele score that is calculated using equation (1) above. Alternatively, other variant callers than the haplotype variant callers can use a read-allele score calculated using equation (2) above. In some implementations, the type of read-allele score used may be determined based on whether the variant calling unit 130 is going to be used to detect SNPs only or SNPs and indels. For example, in some implementations where a variant calling unit will be used to detect SNPs and indels, then the read-allele score calculated using equation (1) above can be used. By way of another example, in other implementations where a variant calling unit will be used to detect only SNPs, then the readl-allele score calculated using equation (2) can be used.

The computer can continue performance of the process 600 by providing one or more inputs to a probability model describing the obtained information describing (i) a read orientation for each of the reads of the pileup of aligned sequence reads stored in the one or more memories, (ii) a position of each base at the reference location with reference to the 5' end of the read for each of the reads of the pileup of aligned sequence reads stored in the one or more memories, (iii) a mapping confidence score for each of the reads, (iv) a read-allele score for each of the reads of the pileup of aligned sequence reads stored in the one or more memories (step 630) for each candidate allele at the reference position, and (v) a base quality score for each read for the base at the reference position such as position "0" (630).

The computer can continue performance of the process 600 by obtaining output information, for each hypothesis of the one or more hypotheses based on the one or more inputs obtained at 620 (640). In the example of the process 600, the obtained input includes inputs for a mapping error probability model and a sequencing error probability model that include (i) a read orientation for each of the reads of the pileup of aligned sequence reads stored in the one or more memories, (ii) a position of each base at the reference location with reference to the 5' end of the read for each of the reads of the pileup of aligned sequence reads stored in the one or more memories, (iii) a mapping confidence score for each of the reads, (iv) a read-allele score for each of the reads of the pileup of aligned sequence reads stored in the one or more memories for each candidate allele at the reference position, and (v) a base quality score for each read for the base at the reference position such as position "0". Accordingly, based on the receipt of these inputs for the mapping error probability model and the sequencing error probability model, the computer will generate output information for one or more hypotheses that account for the occurrence of one or more mapping errors and one or more sequencing errors. Such hypotheses include (i) a likelihood that the reads at the reference position indicate the occurrence of a homozygous ref with a foreign allele that matches the alt, (ii) a likelihood that the reads at the reference position indicate the occurrence of a homozygous alt with a foreign allele that matches the reference allele, (iii) a likelihood that the reads at the reference position indicate the occurrence of a homozygous reference with a sequencing error that matches the alt allele, and (iv) a likelihood that the reads at the reference position indicate the occurrence of a homozygous alt with a sequencing error that matches the reference allele.

The obtained output information includes, for each of these hypotheses, information that is generated by the mapping error probability model and the sequencing error probability model based on each respective probability model's processing of the inputs to the probability models that describe (i) a read orientation for each of the reads of the pileup of aligned sequence reads stored in the one or more memories, (ii) a position of each base at the reference location with reference to the 5' end of the read for each of the reads of the pileup of aligned sequence reads stored in the one or more memories, (iii) a mapping confidence score for each of the reads, (iv) a read-allele score for each of the reads of the pileup of aligned sequence reads stored in the one or more memories for each candidate allele at the reference position, and (v) a base quality score for each read for the base at the reference position such as position "0" (620). In addition, obtained output information includes a score, for each of the particular hypothesis that account for the occurrence of the one or more mapping errors or one or more sequencing errors, that is indicative of a likelihood that the hypothesis is true.

The computer can continue performance of the process 600 by determining, based on the obtained output data generated by the probability model for each of the plurality of hypotheses, a likelihood that a true variant exists at the first position (step 650). In some implementations, this determination may be made, for example, based on an individual, or collective, evaluation of the probability scores determined for each of the one or more hypotheses against one or more predetermined thresholds.

For example, the computer can determine a collective score that is based on the output data generated by the probability model for each of the plurality of hypotheses. The collective score can indicate likelihood that the true variant exists. In some implementations, the collective score can be determined using equation (14). However, other methods of determining the collective score fall within the scope of the present disclosure. If the computer determines, that the collective score satisfies a predetermined threshold, then the computer can add information to a VCF file indicating that a true variant exists at the first position. Alternatively, if the computer determines that the collective score does not satisfy the predetermined threshold, then the computer can discard the output data and determine that the output data indicates the occurrence of a false positive at the reference position.

The processes described with reference to the flowcharts of FIGs. 2, 3, and 5 generally describe processes for correlated error event mitigation for variant calling. These respective processes describe the overarching process for correlated error event mitigation with reference to FIG. 2, a process for mapping error mitigation with reference to FIG. 3, and a process for sequencing error mitigation with reference to FIG. 5. However, when sufficient inputs for accounting for one or more mapping errors and one or more sequencing errors are obtained by the computer, the present disclosure does not require that the separate, disjointed probability calculations for mapping errors and sequencing errors, respectively take place. Instead, in some implementations, a complete probability model may be used in a process such as the process 600 to account for mapping errors and sequencing errors. Though a combined probability model for accounting for mapping errors and sequencing errors will be described below, there is no requirement that the combined probability model be used. Instead, as described with reference to the other implementations, the separate, disjointed probability models may be relied upon.

The description below derives a complete probability calculation for the case of a diploid genome with at most one foreign allele and one systematic error allele. However, this can be directly extended to more alleles in view of the description below. An extended candidate genotype can be defined as $\mathbf{G'}_m = [G_{m,1}\ G_{m,2}\ F_m\ E_m]$. In the expressions below, the notation 0 indicates the REF allele, 1 indicates the first ALT allele, and 2 indicates the second ALT allele, etc. A dash for either $F_m$ or $E_m$ indicates that there is no foreign allele or systematic error allele.

When candidate $\mathbf{G}'_m$ contains no foreign allele or error allele, the following expression results:

$$\Psi_m = P(\mathbf{G}_m) \prod_i \left( \frac{P(r_i \mid G_{m,1}) + P(r_i \mid G_{m,2})}{2} \right).$$   Equation (12)

When $\mathbf{G}'_m$ contains a foreign allele, equation (4) or one of its variations described above can be used. When $\mathbf{G}'_m$ contains an error allele, equation (10) or one of its variations can be used. In general, there is no need to test the case of both a foreign allele and an error allele, because it is extremely rare to find a pileup affected by both of these types of errors at the same time.

As an example of a list of candidates, for the common case of a single ALT allele (REF=0, ALT=1), the following extended candidate genotypes can be tested:

$$\left.\begin{aligned} \mathbf{G}'_1 &= \begin{bmatrix} 0 & 0 & - & - \end{bmatrix} \\ \mathbf{G}'_2 &= \begin{bmatrix} 0 & 1 & - & - \end{bmatrix} \\ \mathbf{G}'_3 &= \begin{bmatrix} 1 & 1 & - & - \end{bmatrix} \end{aligned}\right\} \text{ original hypotheses}$$

$$\left.\begin{aligned} \mathbf{G}'_4 &= \begin{bmatrix} 0 & 0 & 1 & - \end{bmatrix} \\ \mathbf{G}'_5 &= \begin{bmatrix} 1 & 1 & 0 & - \end{bmatrix} \end{aligned}\right\} \text{ foreign read hypotheses}$$

$$\left.\begin{aligned} \mathbf{G}'_6 &= \begin{bmatrix} 0 & 0 & - & 1 \end{bmatrix} \\ \mathbf{G}'_7 &= \begin{bmatrix} 1 & 1 & - & 0 \end{bmatrix} \end{aligned}\right\} \text{ systematic error hypotheses}$$

For each (non-extended) candidate $\mathbf{G}_m$, the joint probability $P(\mathbf{G}_m, R)$ is the maximum over the candidates that match $\mathbf{G}_m$:

$$P(\mathbf{G}_m, R) = \max_{n: G_{n,1} = G_{m,1}, G_{n,2} = G_{m,2}} \Psi_n$$   Equation (14)

and the posterior probability is simply

$$P(\mathbf{G}_m \mid R) = \frac{P(\mathbf{G}_m, R)}{P(R)}$$   Equation (15)

where

$$P(R) = \sum_m P(\mathbf{G}_m, R).$$   Equation (16)

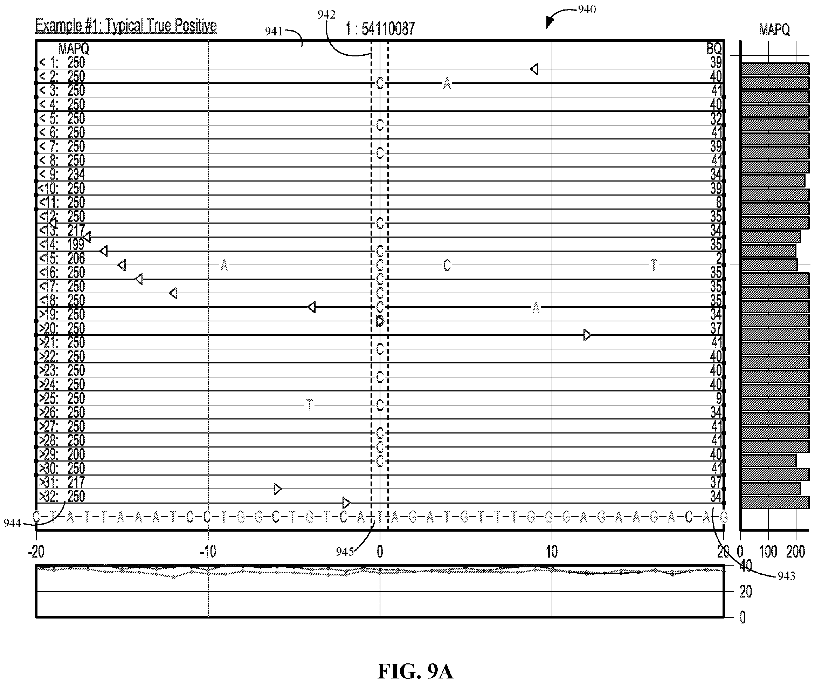

The examples described with reference to FIGs. 9A-12B below illustrate examples of the calculations described with reference to FIGS. 3, 5, and 6 for various pileups.

FIG. 7 is another flowchart of an example of a process 700 for correlated error mitigation for variant calling. The process 700 is described below as being performed by a computer such as the variant calling unit of FIG. 1. In some implementations, the variant calling unit may be implemented using hardware such as one or more FPGAs, ASICs, CPUs, GPUs, or any combination thereof, using software, or any combination thereof.

A computer storing one or more probability models may begin performance of the process 700 by receiving input data that includes information describing one or more characteristics of reads from a pileup of aligned sequence reads (710). In some implementations, the one or more characteristics may include (i) information describing a read orientations for each of the reads of the pileup of aligned sequence reads, (ii) information describing a position of each base at the reference location with reference to the 5' end of the read for each of the reads of the pileup of aligned sequence reads, (iii) a mapping quality score for each of the reads of the pileup of aligned sequence reads, (iv) a read-allele score for each of the reads of the pileup of aligned sequence reads for each candidate allele at the reference position, and (v) a base quality score for each read for the base at the reference position such as position "0". In some implementations, all or a subset of these inputs may be provided as an input to a probability model as with reference other probability models herein. In some implementations, the one or more probability models stored by the computer may include a mapping error probability model and a sequencing error probability model.

In some implementations, the mapping confidence score can include an output of the mapping and aligning unit 126 and can be determined using a phred-scale probability of mapping error $Q_{phred-mapping}$, where $Q_{phred-mapping}$, = -10*log10($P_{e-mapping}$). In this example, $P_{e-mapping}$ is the probability of a mapping error for a particular read. The mapping confidence score 144 value can be proportional to the difference between best alignment score from an aligning algorithm such as a Smith-Waterman aligner and the second best score of the aligner.

The read-allele score may be determined in a number of different ways. For example, a more complex haplotype variant caller can use a read-allele score that is calculated using equation (1) above. Alternatively, other variant callers than the haplotype variant callers can use a read-allele score calculated using equation (2) above. In some implementations, the type of read-allele score used may be determined based on whether the variant calling unit 130 is going to be used to detect SNPs only or SNPs and indels. For example, in some implementations where a variant calling unit will be used to detect SNPs and indels, then the read-allele score calculated using equation (1) above can be used. By way of another example, in other implementations where a variant calling unit will be used to detect only SNPs, then the readl-allele score calculated using equation (2) can be used.

The computer can continue performance of the process 700 by determining a set of one or more hypotheses based on the received inputs (720). For example, if the one or more inputs include information describing read characteristics related to a probability model that accounts for one or more mapping errors, then the computer can determine a set of one or more hypotheses that account for one or more mapping errors. Such hypotheses may include, for example, (i) a likelihood that the reads at the reference position indicate the occurrence of a homozygous ref with a foreign allele that matches the alt and (ii) a likelihood that the reads at the reference position indicate the occurrence of a homozygous alt with a foreign allele that matches the reference allele.

Alternatively, or in addition, for example, if the one or more inputs include information describing read characteristics related to a probability model that accounts for one or more sequencing errors, then the computer can determine a set of one or more hypotheses that account for one or more sequencing errors. Such hypotheses may include (i) a likelihood that the reads at the reference position indicate the occurrence of a homozygous reference with a sequencing error that matches the alt allele and (ii) a likelihood that the reads at the reference position indicate the occurrence of a homozygous alt with a sequencing error that matches the reference allele.

In some implementations, the one or more received inputs may result in the computer determining a set of hypotheses that account for both mapping errors and sequencing errors. Such a determination may be made by the computer when, for example, the one or more received inputs include information describing read characteristics related to a probability model that accounts for both one or more mapping errors and one or more sequencing errors. The set of hypotheses that account for both one or more mapping errors and one or more sequencing errors may include, for example, (i) a likelihood that the reads at the reference position indicate the occurrence of a homozygous ref with a foreign allele that matches the alt, (ii) a likelihood that the reads at the reference position indicate the occurrence of a homozygous alt with a foreign allele that matches the reference allele, (iii) a likelihood that the reads at the reference position indicate the occurrence of a homozygous reference with a sequencing error that matches the alt allele, and (iv) a likelihood that the reads at the reference position indicate the occurrence of a homozygous alt with a sequencing error that matches the reference allele.

The computer can continue performance of the process 700 by determining a score for each hypothesis for each of the one or more hypotheses that indicates a probability that each respective hypotheses determined in stage 720 is true 730. Determining, by the computer, a score for each hypothesis may include, for example, using one or more probability models to determine a probability score for each hypothesis of the one or more hypotheses. An example of a probability model that can be used to determine a score for each mapping error related hypothesis is described with reference to equation (3), described above. However, other variations of other probability models for calculating a score for each mapping error hypothesis are also described above. An example of a probability model that can be used to determine a score for each sequencing error related hypothesis is described with reference to equation (9). However, other variations of other probability models for calculating a score for each sequencing error hypothesis are also described above.

The computer can continue performance of the process 700 by providing output data generated by the probability model that includes the score for each of the one or more hypothesis determined at stage 720 (740). In some implementations, the provided output data can be provided to a second computer that is configured to determine, based on the output data, a likelihood that a true variant exists. In some implementations, the second computer may be a computer that provided the inputs at stage 710. In some implementations, the second computer may be another genomic analysis model, or other computer module, of a secondary analysis unit of which the computer is a part. In some implementations, the second computer may be a remote computer that has a secondary analysis unit with a mapper and aligning module, but no variant call module, that can communicate with the computer using one or more networks.

However, in other implementations, there is no requirement that the computer provide inputs to a second computer. Instead, the output information provided at 740 that includes a set of scores for each of the one or more hypotheses can be used by the computer such as a variant caller, for a determination of whether a candidate allele at a reference position is a true variant or false positive, as described with reference to FIG. 1

## System Components

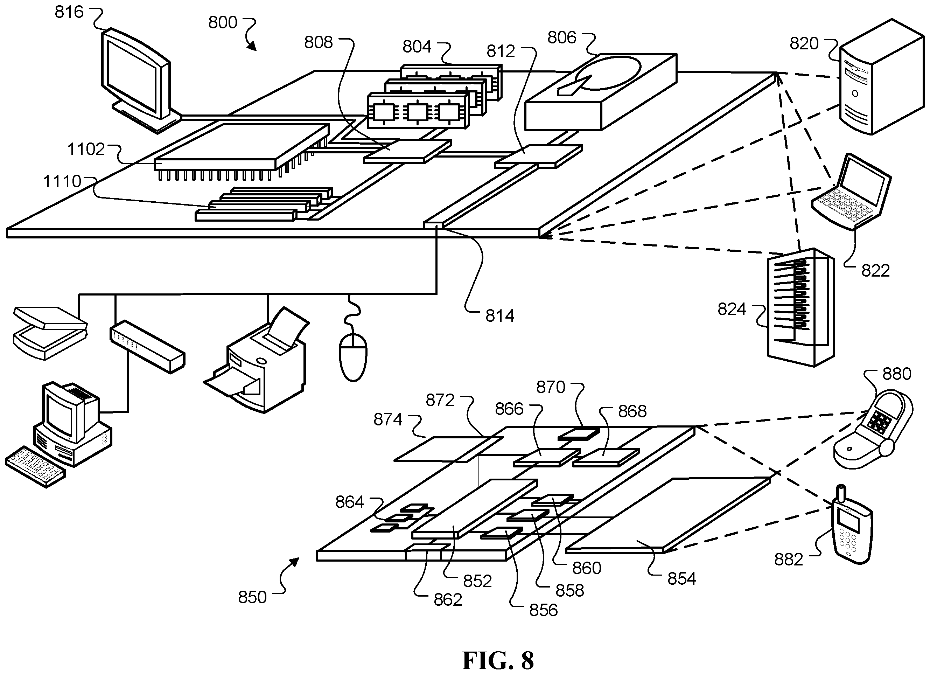

FIG. 8 is a block diagram of system components that can be used to implement a system for correlated error mitigation for variant calling.

Computing device 800 is intended to represent various forms of digital computers, such as laptops, desktops, workstations, personal digital assistants, servers, blade servers, mainframes, and other appropriate computers. Computing device 850 is intended to represent various forms of mobile devices, such as personal digital assistants, cellular telephones, smartphones, and other similar computing devices. Additionally, computing device 800 or 850 can include Universal Serial Bus (USB) flash drives. The USB flash drives can store operating systems and other applications. The USB flash drives can include input/output components, such as a wireless transmitter or USB connector that can be inserted into a USB port of another computing device. The components shown here, their connections and relationships, and their functions, are meant to be exemplary only, and are not meant to limit implementations of the inventions described and/or claimed in this document.

Computing device 800 includes a processor 802, memory 804, a storage device 808, a high-speed interface 808 connecting to memory 804 and high-speed expansion ports 810, and a low speed interface 812 connecting to low speed bus 814 and storage device 808. Each of the components 802, 804, 808, 808, 810, and 812, are interconnected using various busses, and can be mounted on a common motherboard or in other manners as appropriate. The processor 802 can process instructions for execution within the computing device 800, including instructions stored in the memory 804 or on the storage device 808 to display graphical information for a GUI on an external input/output device, such as display 816 coupled to high speed interface 808. In other implementations, multiple processors and/or multiple buses can be used, as appropriate, along with multiple memories and types of memory. Also, multiple computing devices 800 can be connected, with each device providing portions of the necessary operations, e.g., as a server bank, a group of blade servers, or a multi-processor system.

The memory 804 stores information within the computing device 800. In one implementation, the memory 804 is a volatile memory unit or units. In another implementation, the memory 804 is a non-volatile memory unit or units. The memory 804 can also be another form of computer-readable medium, such as a magnetic or optical disk.

The storage device 808 is capable of providing mass storage for the computing device 800. In one implementation, the storage device 808 can be or contain a computer-readable medium, such as a floppy disk device, a hard disk device, an optical disk device, or a tape device, a flash memory or other similar solid state memory device, or an array of devices, including devices in a storage area network or other configurations. A computer program product can be tangibly embodied in an information carrier. The computer program product can also contain instructions that, when executed, perform one or more methods, such as those described above. The information carrier is a computer- or machine-readable medium, such as the memory 804, the storage device 808, or memory on processor 802.

The high speed controller 808 manages bandwidth-intensive operations for the computing device 800, while the low speed controller 812 manages lower bandwidth intensive operations. Such allocation of functions is exemplary only. In one implementation, the high-speed controller 808 is coupled to memory 804, display 816, e.g., through a graphics processor or accelerator, and to high-speed expansion ports 810, which can accept various expansion cards (not shown). In the implementation, low-speed controller 812 is coupled to storage device 808 and low-speed expansion port 814. The low-speed expansion port, which can include various communication ports, e.g., USB, Bluetooth, Ethernet, wireless Ethernet can be coupled to one or more input/output devices, such as a keyboard, a pointing device, microphone/speaker pair, a scanner, or a networking device such as a switch or router, e.g., through a network adapter. The computing device 800 can be implemented in a number of different forms, as shown in the figure. For example, it can be implemented as a standard server 820, or multiple times in a group of such servers. It can also be implemented as part of a rack server system 824. In addition, it can be implemented in a personal computer such as a laptop computer 822. Alternatively, components from computing device 800 can be combined with other components in a mobile device (not shown), such as device 850. Each of such devices can contain one or more of computing device 800, 850, and an entire system can be made up of multiple computing devices 800, 850 communicating with each other.

The computing device 800 can be implemented in a number of different forms, as shown in the figure. For example, it can be implemented as a standard server 820, or multiple times in a group of such servers. It can also be implemented as part of a rack server system 824. In addition, it can be implemented in a personal computer such as a laptop computer 822. Alternatively, components from computing device 800 can be combined with other components in a mobile device (not shown), such as device 850. Each of such devices can contain one or more of computing device 800, 850, and an entire system can be made up of multiple computing devices 800, 850 communicating with each other.

Computing device 850 includes a processor 852, memory 864, and an input/output device such as a display 854, a communication interface 866, and a transceiver 868, among other components. The device 850 can also be provided with a storage device, such as a micro-drive or other device, to provide additional storage. Each of the components 850, 852, 864, 854, 866, and 868, are interconnected using various buses, and several of the components can be mounted on a common motherboard or in other manners as appropriate.

The processor 852 can execute instructions within the computing device 850, including instructions stored in the memory 864. The processor can be implemented as a chipset of chips that include separate and multiple analog and digital processors. Additionally, the processor can be implemented using any of a number of architectures. For example, the processor 810 can be a CISC (Complex Instruction Set Computers) processor, a RISC (Reduced Instruction Set Computer) processor, or a MISC (Minimal Instruction Set Computer) processor. The processor can provide, for example, for coordination of the other components of the device 850, such as control of user interfaces, applications run by device 850, and wireless communication by device 850.

Processor 852 can communicate with a user through control interface 858 and display interface 856 coupled to a display 854. The display 854 can be, for example, a TFT (Thin-Film-Transistor Liquid Crystal Display) display or an OLED (Organic Light Emitting Diode) display, or other appropriate display technology. The display interface 856 can comprise appropriate circuitry for driving the display 854 to present graphical and other information to a user. The control interface 858 can receive commands from a user and convert them for submission to the processor 852. In addition, an external interface 862 can be provide in communication with processor 852, so as to enable near area communication of device 850 with other devices. External interface 862 can provide, for example, for wired communication in some implementations, or for wireless communication in other implementations, and multiple interfaces can also be used.

The memory 864 stores information within the computing device 850. The memory 864 can be implemented as one or more of a computer-readable medium or media, a volatile memory unit or units, or a non-volatile memory unit or units. Expansion memory 874 can also be provided and connected to device 850 through expansion interface 872, which can include, for example, a SIMM (Single In Line Memory Module) card interface. Such expansion memory 874 can provide extra storage space for device 850, or can also store applications or other information for device 850. Specifically, expansion memory 874 can include instructions to carry out or supplement the processes described above, and can include secure information also. Thus, for example, expansion memory 874 can be provide as a security module for device 850, and can be programmed with instructions that permit secure use of device 850. In addition, secure applications can be provided via the SIMM cards, along with additional information, such as placing identifying information on the SIMM card in a non-hackable manner.

The memory can include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 864, expansion memory 874, or memory on processor 852 that can be received, for example, over transceiver 868 or external interface 862.

Device 850 can communicate wirelessly through communication interface 866, which can include digital signal processing circuitry where necessary. Communication interface 866 can provide for communications under various modes or protocols, such as GSM voice calls, SMS, EMS, or MMS messaging, CDMA, TDMA, PDC, WCDMA, CDMA2000, or GPRS, among others. Such communication can occur, for example, through radio-frequency transceiver 868. In addition, short-range communication can occur, such as using a Bluetooth, Wi-Fi, or other such transceiver (not shown). In addition, GPS (Global Positioning System) receiver module 870 can provide additional navigation- and location-related wireless data to device 850, which can be used as appropriate by applications running on device 850.

Device 850 can also communicate audibly using audio codec 860, which can receive spoken information from a user and convert it to usable digital information. Audio codec 860 can likewise generate audible sound for a user, such as through a speaker, e.g., in a handset of device 850. Such sound can include sound from voice telephone calls, can include recorded sound, e.g., voice messages, music files, etc. and can also include sound generated by applications operating on device 850.

The computing device 850 can be implemented in a number of different forms, as shown in the figure. For example, it can be implemented as a cellular telephone 880. It can also be implemented as part of a smartphone 882, personal digital assistant, or other similar mobile device.

Various implementations of the systems and methods described here can be realized in digital electronic circuitry, integrated circuitry, specially designed ASICs (application specific integrated circuits), computer hardware, firmware, software, and/or combinations of such implementations. These various implementations can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" "computer-readable medium" refers to any computer program product, apparatus and/or device, e.g., magnetic discs, optical disks, memory, Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device, e.g., a CRT (cathode ray tube) or LCD (liquid crystal display) monitor for displaying information to the user and a keyboard and a pointing device, e.g., a mouse or a trackball by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input.

The systems and techniques described here can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the systems and techniques described here, or any combination of such back end, middleware, or front end components. The components of the system can be interconnected by any form or medium of digital data communication, e.g., a communication network. Examples of communication networks include a local area network ("LAN"), a wide area network ("WAN"), and the Internet.

The computing system can include clients and servers. A client and server are generally remote from each other and typically interact through a communication network. The relationship of client and server arises by virtue of computer programs running on the respective computers and having a client-server relationship to each other.

A number of embodiments have been described. Nevertheless, it will be understood that various modifications can be made without departing from the spirit and scope of the invention. In addition, the logic flows depicted in the figures do not require the particular order shown, or sequential order, to achieve desirable results. In addition, other steps can be provided, or steps can be eliminated, from the described flows, and other components can be added to, or removed from, the described systems. Accordingly, other embodiments are within the scope of the following claims.

## EXAMPLES

The subject matter of the present disclosure is further described with reference to the following examples, which do not limit the scope of the present disclosure in any way.

The examples provided in this section show real-world examples that illustrate calculations using the probability models described herein on real-world pileups. Each pileup plot includes a MAPQ mapping confidence score per read, a base quality at the reference position such as position "0," and the mean base quality per strand (blue = forward, red = reverse).

### Example 1 - Typical True Positive Variant

FIG. 9A is an example of summary of experimental results from execution of a process for correlated error mitigation for variant calling that has been performed on a pileup of sequencing reads whose result indicates an example of a true positive results.

In more detail, FIG. 9A displays an image 940 of a pileup 941 of aligned sequence reads showing a typical true positive example. A true positive is an example of a pileup 941 of reads having a true variant at the reference location 942. In this example, it can be seen that the read characteristics indicate that there is a candidate alt allele "C" at reference position 942 that differs from the reference "T" of the reference genome to which the pileup 941 of reads is mapped and aligned.

In this example, and with reference to the image 940 of the pileup 941 of FIG. 9A, it can be seen that the alt allele frequency for "C" is balanced between the first forward read direction (or orientation) and the second reverse read direction (or orientation), the MAPQ mapping confidence score is high for each of the reads at the reference location with most of the mapping confidence scores 944 at the maximum of "250," and the mean base quality in both strand directions (or orientations) is high with most of the base quality scores 943 at "35" or above. As a result, the probability score for the candidate [101-1-] 962 is high, as shown in the probability score result column 980 and normalized probability score result column 980. Accordingly, information identifying, or otherwise associated with, the true alt "C" can be included in the VCF file.

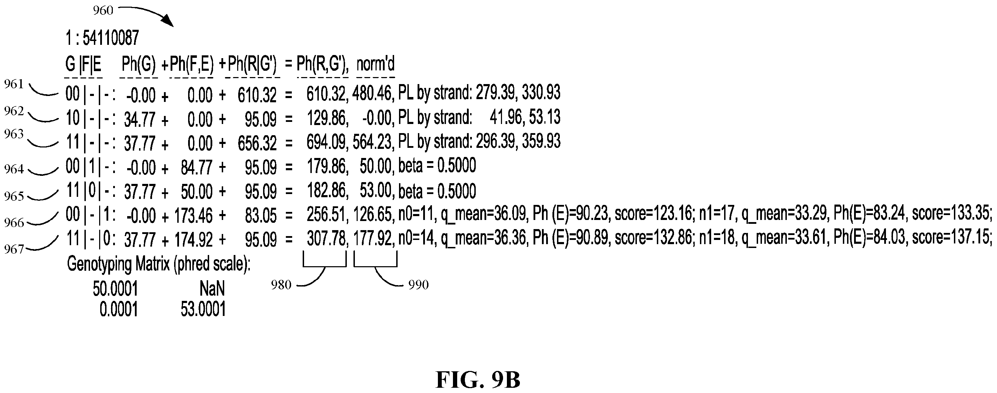

The complete set of modified probability results 960 is shown in FIG. 9B. The candidate [10|-|-] 962 corresponds to hypothesis, 162, above which includes a likelihood that the reads at the reference position 142 indicate the occurrence of a heterozygous alt allele. The probability score result column 980 and the normalized probability score result column 990 show the probability score result and normalized score result, respectively, for each of the other conventional hypotheses 961, 962, 963 and non-conventional hypotheses 964, 965, 966, 967. For purposes of clarity, the conventional hypothesis 961 corresponds to the conventional hypothesis 161 above, the conventional hypothesis 962 corresponds to the conventional hypothesis 162 above, and the conventional hypothesis 963 corresponds to the conventional hypothesis 163 above. In addition, the non-conventional hypothesis 964 corresponds to the non-conventional hypothesis 164 above, non-conventional hypothesis 965 corresponds to the non-conventional hypothesis 165 above, non-conventional hypothesis 966 corresponds to the non-conventional hypothesis 166 above, and the non-conventional hypothesis 967 corresponds to the non-conventional hypothesis 167 above.

Example 2 - Low Likelihood of Mapping Error

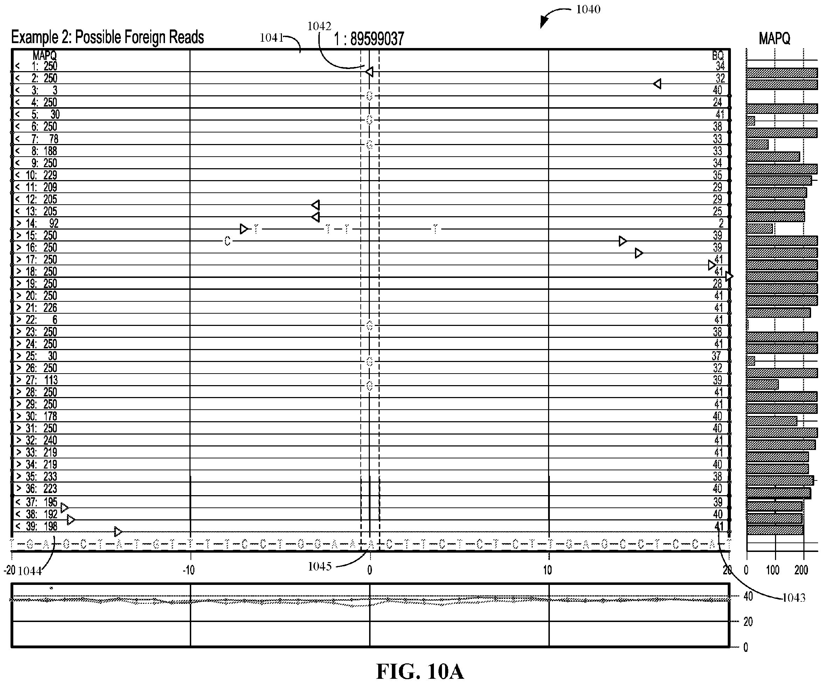

FIG. 10A is an example of summary of experimental results from execution of a process for correlated error mitigation for variant calling that has been performed on a pileup of sequencing reads whose result indicates a low likelihood of an occurrence of a mapping error.

In more detail, FIG. 10A displays an image 1040 of a pileup 1041 of aligned sequence reads showing a possible foreign read, or possible mapping error, example. In this example, the pileup has a low alt allele frequency and moderately low MAPQ mapping confidence scores 1044 for reads having the alt alleles. Both of these factors would be exploited by the mapping error probability models provided by the present disclosure. The resulting likelihood that this represents a true variant is therefore diminished. A review of the probabilities scores for each respective hypothesis indicates that a conclusion cannot be drawn with high confidence that the "C" alleles are foreign reads, nor can a decision with high confidence be made in a heterozygous call. Accordingly, the output information may be discarded without adding any information to the VCF file identifying the candidate alt allele.

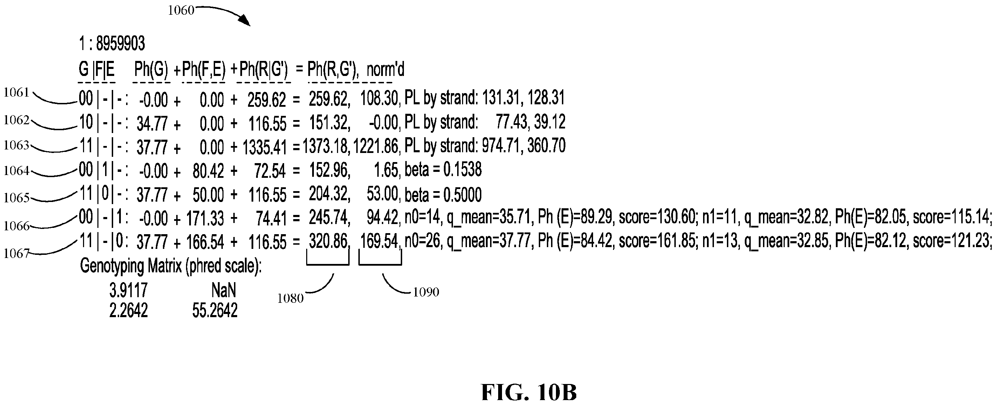

The complete set of modified probability results 1060 is shown in FIG. 10B. The probability score result column 1080 and the normalized probability score result column 1090 show the probability score result and normalized score result, respectively, for each of the other conventional hypotheses 1061, 1062, 1063 and non-conventional hypotheses 1064, 1065, 1066, 1067. For purposes of clarity, the conventional hypothesis 1061 corresponds to the conventional hypothesis 161 above, the conventional hypothesis 1062 corresponds to the conventional hypothesis 162 above, and the conventional hypothesis 1063 corresponds to the conventional hypothesis 163 above. In addition, the non-conventional hypothesis 1064 corresponds to the non-conventional hypothesis 164 above, non-conventional hypothesis 1065 corresponds to the non-conventional hypothesis 165 above, non-conventional hypothesis 1066 corresponds to the non-conventional hypothesis 166 above, and the non-conventional hypothesis 1067 corresponds to the non-conventional hypothesis 167 above.

Example 3 - Unlikely True Variant Due to Sequencing Error

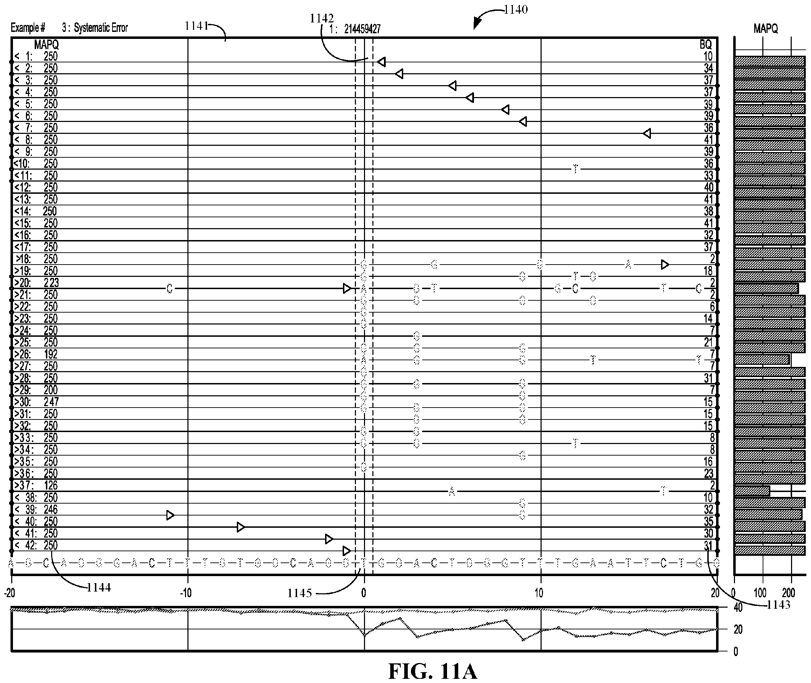

FIG. 11A is an example of summary of experimental results from execution of a process for correlated error mitigation for variant calling that has been performed on a pileup of sequencing reads whose result indicates a high likelihood that the candidate alt allele is unlikely to be a true variant due to a sequencing error.

In more detail, FIG. 11A displays an image 1140 of a pileup 1141 of aligned sequence reads showing a systematic error, or sequencing error.

In this example, all of the "G" alt alleles occur in a single read orientation in the forward aligned direction. In addition, all of the "G" alt alleles occur on a subset of the forward oriented reads that are furthest from the 5' end of the read. The subset of reads having the "G" alt alleles has very low base quality as evidenced by the base quality scores 1143. In addition, the "G" alt allele matches the two base alleles of the reference genome that present the base allele at the current reference position 1145. The sequencing error probability model takes the aforementioned characteristics of the reads into account and the probability scores output for each of the seven hypotheses 1161, 1162, 1163, 1164, 1165, 1166, 1167 and shown in the probability score result 1180 and the normalized probability score result column 1190, support, with high confidence, that the "G" alt allele is unlikely to support a true variant. Accordingly, the output information may be discarded without adding any information to the VCF file identifying the candidate alt allele.

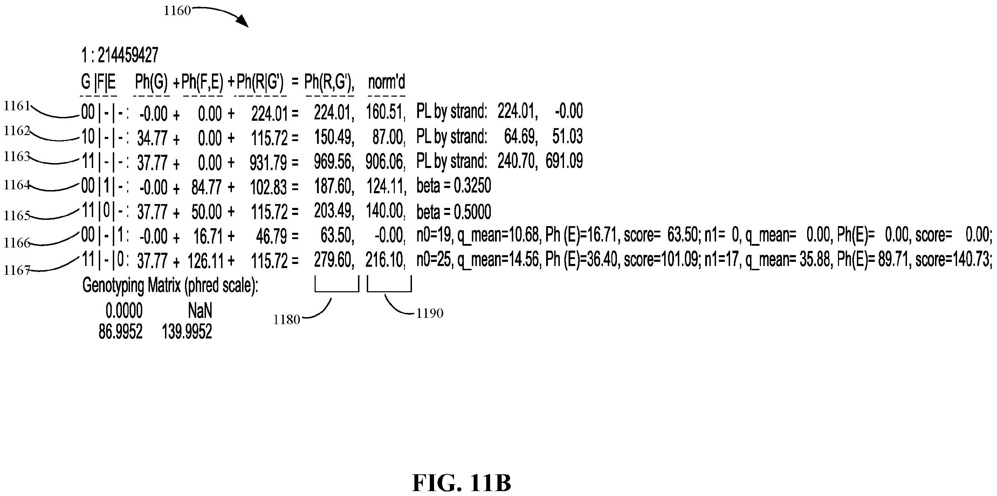

The complete set of modified probability results 1160 is shown in FIG. 11B. The probability score result column 1180 and the normalized probability score result column 1190 show the probability score result and normalized score result, respectively, for each of the other conventional hypotheses 1161, 1162, 1163 and non-conventional hypotheses 1164, 1165, 1166, 1167. For purposes of clarity, the conventional hypothesis 1161 corresponds to the conventional hypothesis 161 above, the conventional hypothesis 1162 corresponds to the conventional hypothesis 162 above, and the conventional hypothesis 1163 corresponds to the conventional hypothesis 163 above. In addition, the non-conventional hypothesis 1164 corresponds to the non-conventional hypothesis 164 above, non-conventional hypothesis 1165 corresponds to the non-conventional hypothesis 165 above, non-conventional hypothesis 1166 corresponds to the non-conventional hypothesis 166 above, and the non-conventional hypothesis 1167 corresponds to the non-conventional hypothesis 167 above.

Example 4 - Unlikely True Variant Due to Sequencing Error In Both Read Orientations

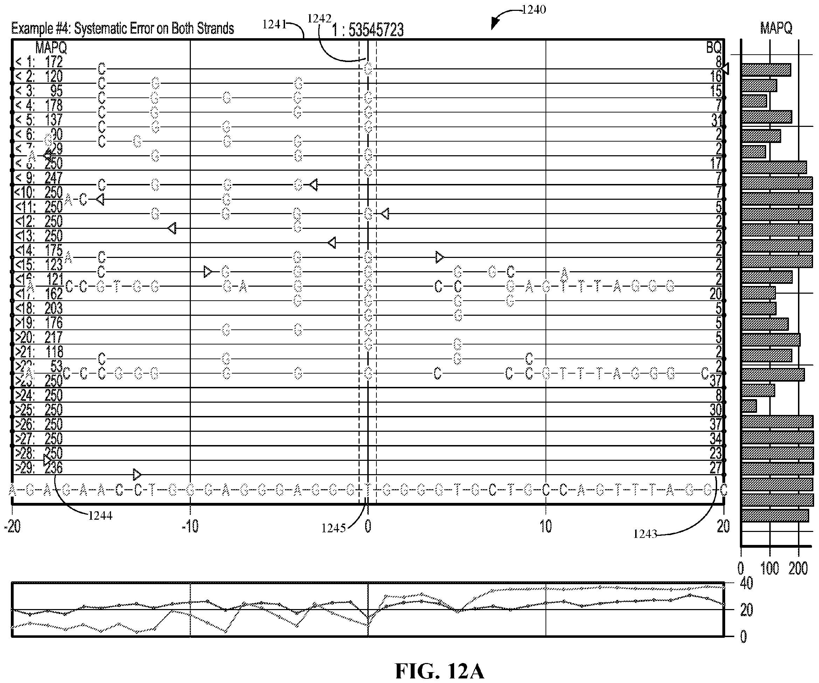

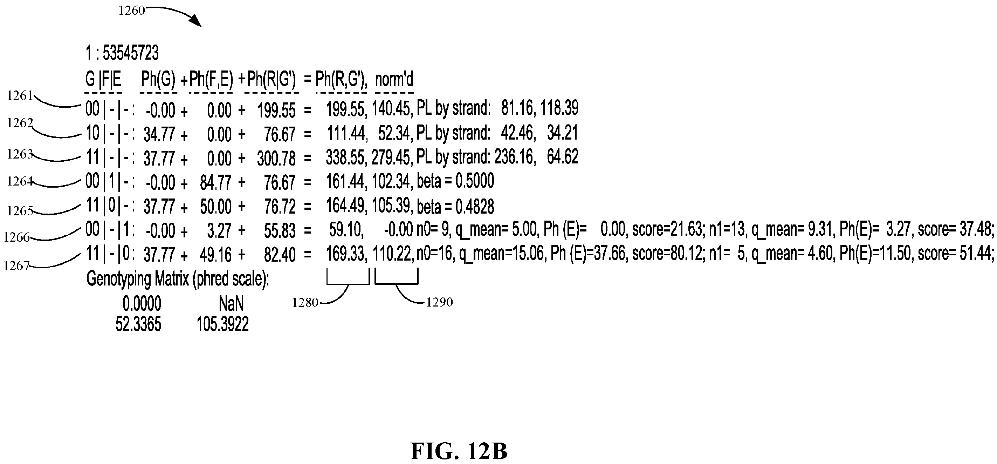

FIG. 12A is an example of summary of experimental results from execution of a process for correlated error mitigation for variant calling that has been performed on a pileup of sequencing reads whose result indicates a high likelihood that the candidate alt allele is unlikely to be a true variant due to a sequencing error on both read orientations.
In more detail, FIG. 12A displays an image 1240 of a pileup 1241 of aligned sequence reads showing a systematic error, or sequencing error, on both read orientations.
In this example, there is an extreme drop off in base quality as evidenced by the base quality scores 1243. On the forward aligned reads, the "G" alt alleles are confined to a subset of reads where the locus of interest is furthers from the 5' end. In addition, the "G" alt allele matches the preceding three reference alleles of the reference genome that precede the reference allele at the reference position 1242. The sequencing error probability model takes the aforementioned characteristics of the reads into account and the probability scores output for each of the seven hypotheses 1261, 1262, 1263, 1264, 1265, 1266, 1267 and shown in the probability score result 1280 and the normalized probability score result column 1290, support, with high confidence, that the "G" alt allele is unlikely to support a true variant. Accordingly, the output information may be discarded without adding any information to the VCF file identifying the candidate alt allele.
The complete set of modified probability results 1260 is shown in FIG. 12B. The probability score result column 1280 and the normalized probability score result column 1290 show the probability score result and normalized score result, respectively, for each of the other conventional hypotheses 1161, 1262, 1263 and non-conventional hypotheses 1264, 1265, 1266, 1267. For purposes of clarity, the conventional hypothesis 1261 corresponds to the conventional hypothesis 161 above, the conventional hypothesis 1262 corresponds to the conventional hypothesis 162 above, and the conventional hypothesis 1263 corresponds to the conventional hypothesis 163 above. In addition, the non-conventional hypothesis 1264 corresponds to the non-conventional hypothesis 164 above, non-conventional hypothesis 1265 corresponds to the non-conventional hypothesis 165 above, non-conventional hypothesis 1266 corresponds to the non-conventional hypothesis 166 above, and the non-conventional hypothesis 1267 corresponds to the non-conventional hypothesis 167 above.

## OTHER EMBODIMENTS

It is to be understood that while the invention has been described in conjunction with the drawings and detailed description thereof, the foregoing description is intended to illustrate and not limit the scope of the invention, which is defined by the scope of the appended claims. Other aspects, advantages, and modifications are within the scope of the claims.

## FURTHER EMBODIMENTS

Although the present invention is defined in the claims, it should be understood that the present invention can also (alternatively) be defined in accordance with the following embodiments:

1. A method for improving the accuracy of a variant call by accounting for indications of correlated error events, the method comprising:

accessing, by one or more computers and from one or more memory devices, a pileup of a plurality of sequence reads aligned to a first region of a reference genome;
obtaining, by the one or more computers, information describing one or more characteristics of each of the plurality of reads of the pileup corresponding to a first position of the reference genome;
providing, by the one or more computers and based on the obtained information, one or more inputs to a probability model describing the one or more characteristics of the plurality of reads of the pileup, wherein the probability model is configured to determine a score, for each hypothesis of one or more hypotheses selected based on the one or more inputs, that indicates whether the hypothesis is true;
obtaining, by the one or more computers, output information for each of the one or more hypotheses, wherein the output information for each of the one or more hypotheses is (i) generated by the probability model based on the probability model's processing of the one or more inputs to the probability model describing the one or more characteristics of the respective reads of the pileup and (ii) indicative of a score that indicates whether the hypothesis is true; and

determining, by the one or more computers and based on the obtained output information generated by the probability model for each of the plurality of hypotheses, a likelihood that a true variant exists at the first position.

2. The method of embodiment 1, wherein determining, by the one or more computers and based on the obtained output information generated by the probability model for each of the plurality of hypotheses, a likelihood that a true variant exists at the first position comprises:

determining, by the one or more computers, a collective score that is based on the output information generated by the probability model for each of the plurality of hypothesis, wherein the collective score indicates a likelihood that the true variant exists;

determining, by the one or more computers, whether the score generated by the collective score satisfies a predetermined threshold; and

based on determining, by the one or more computers, that the collective score satisfies the predetermined threshold, adding information to a VCF file indicating that a true variant exists at the first position.

3. The method of embodiment 2, wherein the information indicating that a true variant exists at the first position includes information identifying (i) the first position, (ii) a candidate alt allele at the first position, (iii) the collective score.

4. The method of embodiment 1, wherein the information describing the one or more characteristics of the respective reads includes information describing (i) a mapping quality score for each sequence read in the pileup at the first position and (ii) a read-allele score for each sequence read in the pileup at the first position for each candidate allele at the first position.

5. The method of embodiment 4, wherein the read-allele score for each read of the pileup at the first position is based on an output produced by a P-HMM model, for each of the reads at the first position, which indicates a probability of observing a read $r_i$ given a particular candidate allele $G_{m,\phi}$.

6. The method of embodiment 4, wherein the output information includes:

first output information for a first hypothesis of the one or more hypotheses that includes a likelihood that the sequence reads at the first position indicate the occurrence of a homozygous reference with a foreign allele that matches the alt; and

second output information for a second hypothesis of the one or more hypotheses that includes a likelihood that the sequence reads at the first position indicate the occurrence of a homozygous alt with a foreign allele that matches a reference allele.

7. The method of embodiment 1, wherein the information describing the one or more characteristics of the respective sequence reads includes information describing (i) a read orientation for each sequence read of the pileup at the first position, (ii) a position of each base at the first position within each sequence read of the pileup at the first position with reference to the 5' end of the sequence read, (iii) a read-allele score for each sequence read of the plurality of sequence reads for each candidate allele at the reference position, and (iv) a base quality score for each read for the base at the first position.

8. The method of embodiment 7, wherein the read-allele score for each sequence read of the pileup at the first position is based on an output produced by a P-HMM model, for each of the sequence reads at the first position, that indicates a probability of observing a sequence read $r_i$ given a particular candidate allele $G_{m,\phi}$.

9. The method of embodiment 1, wherein the information describing the one or more characteristics of the respective sequence reads includes information describing (i) a read orientation for each sequence read of the pileup at the first position, (ii) a position of each base at the first position within each sequence read of the pileup at the first position with reference to the 5' end of the sequence read, and (iii) a read-allele score for each sequence read of the plurality of sequence reads for each candidate allele at the first position.

10. The method of embodiment 7, wherein the output information includes:

first output information for a first hypothesis of the one or more hypotheses that includes a likelihood that the sequence reads at the first position indicate the occurrence of a homozygous reference with a sequencing error that matches the alt allele, and

second output information for a second hypothesis of the one or more hypotheses that includes a likelihood that the sequence reads at the first position indicate the occurrence of a homozygous alt with a sequencing error that matches the reference allele.

11. The method of embodiment 1, wherein the information describing the one or more characteristics of the respective sequence reads includes information describing (i) a read orientation for each sequence read of the pileup at the first position, (ii) a position of each base at the first position such as position "0" 142 within each sequence read with reference to the 5' end of the sequence read, (iii) a mapping quality score for each sequence read of the pileup at the first position, (iv) a read-allele score for each sequence read of the plurality of reads for each candidate allele at the reference position, and (v) a base quality score for each read for the base aligned at the first position.

12. The method of embodiment 11, wherein the read-allele score for each sequence read of the pileup at the first position is based on an output produced by a P-HMM model, for each of the sequence reads at the first position, that indicates a probability of observing a read $r_i$ given a particular candidate allele $G_{m,\phi}$.

13. The method of embodiment 11, wherein the output information includes:

a first likelihood that the sequence reads at the first position indicate the occurrence of a homozygous reference with a foreign allele that matches the alt,

a second likelihood that the sequence reads at the first position indicate the occurrence of a homozygous alt with a foreign allele that matches a reference allele,

a third output information for a first hypothesis of the one or more hypotheses that includes a likelihood that the sequence reads at the first position indicate the occurrence of a homozygous reference with a sequencing error that matches the alt allele, and

a fourth output information for a second hypothesis of the one or more hypotheses that includes a likelihood that the sequence reads at the first position indicate the occurrence of a homozygous alt with a sequencing error that matches the reference allele.

14. The method of embodiment 1, wherein the one or more memory devices received the pileup of aligned sequence reads from a Field Programmable Gate Array (FPGA) device, wherein the FPGA includes one or more configurable digital logic gates that have been configured as a mapping and alignment unit to perform read mapping and alignment.

15. The method of embodiment 14,

wherein the computer is configured to access the one or more memory devices using one or more wired or wireless networks,

wherein a Field Programmable Gate Array (FPGA) device and the one or more memory devices are housed in an expansion card that has been coupled to a circuit board of a sequencer,

wherein the sequencer is configured to generate sequence reads based on an input sample and store the generated sequence reads in the one or more memory devices, and

wherein the mapping and alignment unit of the FPGA is configured to access the one or more memory devices to obtain the generated sequence reads.

16. The method of embodiment 14,

wherein the computer and the sequencer are each configured to access the one or more memory devices using one or more wired or wireless networks,

wherein the Field Programmable Gate Array (FPGA) device and the one or more memory devices are housed in an expansion card that has been coupled to a circuit board of server that is located remotely from the computer and the sequencer,

wherein the sequencer is configured to generate sequence reads based on an input sample, provide the generated sequence reads to the server using the one or more wired or wireless networks for storage, of the generated sequence reads, in the one or more memory devices, and

wherein the mapping and alignment unit of the FPGA is configured to access the one or more memory devices to obtain the generated sequence reads.

17. A system comprising:
one or more computers and one or more storage devices storing instructions that are operable, when executed by the one or more computers, to cause the one or more computers to perform operations comprising:

accessing, by one or more computers and from one or more memory devices, a pileup of a plurality of sequence reads aligned to a first region of a reference genome;

obtaining, by the one or more computers, information describing one or more characteristics of each of the plurality of reads of the pileup corresponding to a first position of the reference genome;

providing, by the one or more computers and based on the obtained information, one or more inputs to a probability model describing the one or more characteristics of the plurality of reads of the pileup, wherein the probability model is configured to determine a score, for each hypothesis of one or more hypotheses selected based on the one or more inputs, that indicates whether the hypothesis is true;

obtaining, by the one or more computers, output information for each of the one or more hypotheses, wherein the output information for each of the one or more hypotheses is (i) generated by the probability model based on the probability model's processing of the one or more inputs to the probability model describing the one or more characteristics of the respective reads of the pileup and (ii) indicative of a score that indicates whether the hypothesis is true; and

determining, by the one or more computers and based on the obtained output information generated by the probability model for each of the plurality of hypotheses, a likelihood that a true variant exists at the first position.

18. The system of embodiment 19, wherein determining, by the one or more computers and based on the obtained output information generated by the probability model for each of the plurality of hypotheses, a likelihood that a true variant exists at the first position comprises:

determining, by the one or more computers, a collective score that is based on the output information generated by the probability model for each of the plurality of hypothesis, wherein the collective score indicates a likelihood that the true variant exists;

determining, by the one or more computers, whether the score generated by the collective score satisfies a predetermined threshold; and

based on determining, by the one or more computers, that the collective score satisfies the predetermined threshold, adding information to a VCF file indicating that a true variant exists at the first position.

19. The system of embodiment 18, wherein the information indicating that a true variant exists at the first position includes information identifying (i) the first position, (ii) a candidate alt allele at the first position, (iii) the collective score.

20. The system of embodiment 17, wherein the information describing the one or more characteristics of the respective reads includes information describing (i) a mapping quality score for each sequence read in the pileup at the first position and (ii) a read-allele score for each sequence read in the pileup at the first position for each candidate allele at the first position.

21. The system of embodiment 20, wherein the read-allele score for each read of the pileup at the first position is based on an output produced by a P-HMM model, for each of the reads at the first position, which indicates a probability of observing a read $r_i$ given a particular candidate allele $G_{m,\phi}$.

22. The system of embodiment 20, wherein the output information includes:

first output information for a first hypothesis of the one or more hypotheses that includes a likelihood that the sequence reads at the first position indicate the occurrence of a homozygous reference with a foreign allele that matches the alt; and

second output information for a second hypothesis of the one or more hypotheses that includes a likelihood that the sequence reads at the first position indicate the occurrence of a homozygous alt with a foreign allele that matches a reference allele.

23. The system of embodiment 17, wherein the information describing the one or more characteristics of the respective sequence reads includes information describing (i) a read orientation for each sequence read of the pileup at the first position, (ii) a position of each base at the first position within each sequence read of the pileup at the first position with reference to the 5' end of the sequence read, (iii) a read-allele score for each sequence read of the plurality of sequence reads for each candidate allele at the reference position, and (iv) a base quality score for each read for the base at the first position.

24. The system of embodiment 23, wherein the read-allele score for each sequence read of the pileup at the first position is based on an output produced by a P-HMM model, for each of the sequence reads at the first position, that indicates a probability of observing a sequence read $r_i$ given a particular candidate allele $G_{m,\phi}$.

25. The system of embodiment 17, wherein the information describing the one or more characteristics of the respective sequence reads includes information describing (i) a read orientation for each sequence read of the pileup at the first position, (ii) a position of each base at the first position within each sequence read of the pileup at the first position with reference to the 5' end of the sequence read, and (iii) a read-allele score for each sequence read of the plurality of sequence reads for each candidate allele at the first position.

26. The system of embodiment 25, wherein the output information includes:

first output information for a first hypothesis of the one or more hypotheses that includes a likelihood that the sequence reads at the first position indicate the occurrence of a homozygous reference with a sequencing error that matches the alt allele, and
second output information for a second hypothesis of the one or more hypotheses that includes a likelihood that the sequence reads at the first position indicate the occurrence of a homozygous alt with a sequencing error that matches the reference allele.

27. The system of embodiment 17, wherein the information describing the one or more characteristics of the respective sequence reads includes information describing (i) a read orientation for each sequence read of the pileup at the first position, (ii) a position of each base at the first position such as position "0" 142 within each sequence read with reference to the 5' end of the sequence read, (iii) a mapping quality score for each sequence read of the pileup at the first position, (iv) a read-allele score for each sequence read of the plurality of reads for each candidate allele at the reference position, and (v) a base quality score for each read for the base aligned at the first position.

28. The system of embodiment 27, wherein the read-allele score for each sequence read of the pileup at the first position is based on an output produced by a P-HMM model, for each of the sequence reads at the first position, that indicates a probability of observing a read $r_i$ given a particular candidate allele $G_{m,\phi}$.

29. The system of embodiment 27, wherein the output information includes:

a first likelihood that the sequence reads at the first position indicate the occurrence of a homozygous reference with a foreign allele that matches the alt,
a second likelihood that the sequence reads at the first position indicate the occurrence of a homozygous alt with a foreign allele that matches a reference allele,
a third output information for a first hypothesis of the one or more hypotheses that includes a likelihood that the sequence reads at the first position indicate the occurrence of a homozygous reference with a sequencing error that matches the alt allele, and
a fourth output information for a second hypothesis of the one or more hypotheses that includes a likelihood that the sequence reads at the first position indicate the occurrence of a homozygous alt with a sequencing error that matches the reference allele.

30. The system of embodiment 17, wherein the one or more memory devices received the pileup of aligned sequence reads from a Field Programmable Gate Array (FPGA) device, wherein the FPGA includes one or more configurable digital logic gates that have been configured as a mapping and alignment unit to perform read mapping and alignment.

31. The system of embodiment 30,

wherein the computer is configured to access the one or more memory devices using one or more wired or wireless networks,
wherein a Field Programmable Gate Array (FPGA) device and the one or more memory devices are housed in an expansion card that has been coupled to a circuit board of a sequencer,
wherein the sequencer is configured to generate sequence reads based on an input sample and store the generated sequence reads in the one or more memory devices, and
wherein the mapping and alignment unit of the FPGA is configured to access the one or more memory devices to obtain the generated sequence reads.

32. The system of embodiment 30,

wherein the computer and the sequencer are each configured to access the one or more memory devices using one or more wired or wireless networks,
wherein the Field Programmable Gate Array (FPGA) device and the one or more memory devices are housed in an expansion card that has been coupled to a circuit board of server that is located remotely from the computer and the sequencer,
wherein the sequencer is configured to generate sequence reads based on an input sample, provide the generated sequence reads to the server using the one or more wired or wireless networks for storage, of the generated sequence reads, in the one or more memory devices, and
wherein the mapping and alignment unit of the FPGA is configured to access the one or more memory devices to obtain the generated sequence reads.

33. A non-transitory computer-readable medium storing software comprising instructions executable by one or more computers which, upon such execution, cause the one or more computers to perform operations comprising:

accessing, by the one or more computers and from one or more memory devices, a pileup of a plurality of sequence reads aligned to a first region of a reference genome;
obtaining, by the one or more computers, information describing one or more characteristics of each of the plurality of reads of the pileup corresponding to a first position of the reference genome;
providing, by the one or more computers and based on the obtained information, one or more inputs to a probability model describing the one or more characteristics of the plurality of reads of the pileup, wherein the probability model is configured to determine a score, for each hypothesis of one or more hypotheses selected based on the one or more inputs, that indicates whether the hypothesis is true;
obtaining, by the one or more computers, output information for each of the one or more hypotheses, wherein the output information for each of the one or more hypotheses is (i) generated by the probability model based on the probability model's processing of the one or more inputs to the probability model describing the one or more characteristics of the respective reads of the pileup and (ii) indicative of a score that indicates whether the hypothesis is true; and
determining, by the one or more computers and based on the obtained output information generated by the probability model for each of the plurality of hypotheses, a likelihood that a true variant exists at the first position.

34. The computer-readable medium of embodiment 33, wherein determining, by the one or more computers and based on the obtained output information generated by the probability model for each of the plurality of hypotheses, a likelihood that a true variant exists at the first position comprises:

determining, by the one or more computers, a collective score that is based on the output information generated by the probability model for each of the plurality of hypothesis, wherein the collective score indicates a likelihood that the true variant exists;
determining, by the one or more computers, whether the score generated by the collective score satisfies a predetermined threshold; and
based on determining, by the one or more computers, that the collective score satisfies the predetermined threshold, adding information to a VCF file indicating that a true variant exists at the first position.

35. The computer-readable medium of embodiment 34, wherein the information indicating that a true variant exists at the first position includes information identifying (i) the first position, (ii) a candidate alt allele at the first position, (iii) the collective score.

36. The computer-readable medium of embodiment 33, wherein the information describing the one or more characteristics of the respective reads includes information describing (i) a mapping quality score for each sequence read in the pileup at the first position and (ii) a read-allele score for each sequence read in the pileup at the first position for each candidate allele at the first position.

37. The computer-readable medium of embodiment 36, wherein the read-allele score for each read of the pileup at the first position is based on an output produced by a P-HMM model, for each of the reads at the first position, which indicates a probability of observing a read $r_i$ given a particular candidate allele $G_{m,\phi}$.

38. The computer-readable medium of embodiment 36, wherein the output information includes:

first output information for a first hypothesis of the one or more hypotheses that includes a likelihood that the sequence reads at the first position indicate the occurrence of a homozygous reference with a foreign allele that matches the alt; and

second output information for a second hypothesis of the one or more hypotheses that includes a likelihood that the sequence reads at the first position indicate the occurrence of a homozygous alt with a foreign allele that matches a reference allele.

39. The computer-readable medium of embodiment 33, wherein the information describing the one or more characteristics of the respective sequence reads includes information describing (i) a read orientation for each sequence read of the pileup at the first position, (ii) a position of each base at the first position within each sequence read of the pileup at the first position with reference to the 5' end of the sequence read, (iii) a read-allele score for each sequence read of the plurality of sequence reads for each candidate allele at the reference position, and (iv) a base quality score for each read for the base at the first position.

40. The computer-readable medium of embodiment 39, wherein the read-allele score for each sequence read of the pileup at the first position is based on an output produced by a P-HMM model, for each of the sequence reads at the first position, that indicates a probability of observing a sequence read $r_i$ given a particular candidate allele $G_{m,\phi}$.

41. The computer-readable medium of embodiment 39, wherein the information describing the one or more characteristics of the respective sequence reads includes information describing (i) a read orientation for each sequence read of the pileup at the first position, (ii) a position of each base at the first position within each sequence read of the pileup at the first position with reference to the 5' end of the sequence read, and (iii) a read-allele score for each sequence read of the plurality of sequence reads for each candidate allele at the first position.

42. The computer-readable medium of embodiment 39, wherein the output information includes:

first output information for a first hypothesis of the one or more hypotheses that includes a likelihood that the sequence reads at the first position indicate the occurrence of a homozygous reference with a sequencing error that matches the alt allele, and

second output information for a second hypothesis of the one or more hypotheses that includes a likelihood that the sequence reads at the first position indicate the occurrence of a homozygous alt with a sequencing error that matches the reference allele.

43. The computer-readable medium of embodiment 33, wherein the information describing the one or more characteristics of the respective sequence reads includes information describing (i) a read orientation for each sequence read of the pileup at the first position, (ii) a position of each base at the first position such as position "0" 142 within each sequence read with reference to the 5' end of the sequence read, (iii) a mapping quality score for each sequence read of the pileup at the first position, (iv) a read-allele score for each sequence read of the plurality of reads for each candidate allele at the reference position, and (v) a base quality score for each read for the base aligned at the first position.

44. The computer-readable medium of embodiment 43, wherein the read-allele score for each sequence read of the pileup at the first position is based on an output produced by a P-HMM model, for each of the sequence reads at the first position, that indicates a probability of observing a read $r_i$ given a particular candidate allele $G_{m,\phi}$.

45. The computer-readable medium of embodiment 43, wherein the output information includes:

a first likelihood that the sequence reads at the first position indicate the occurrence of a homozygous reference with a foreign allele that matches the alt,

a second likelihood that the sequence reads at the first position indicate the occurrence of a homozygous alt with a foreign allele that matches a reference allele,

a third output information for a first hypothesis of the one or more hypotheses that includes a likelihood that the sequence reads at the first position indicate the occurrence of a homozygous reference with a sequencing error that matches the alt allele, and

a fourth output information for a second hypothesis of the one or more hypotheses that includes a likelihood that the sequence reads at the first position indicate the occurrence of a homozygous alt with a sequencing error that matches the reference allele.

46. The computer-readable medium of embodiment 33, wherein the one or more memory devices received the pileup of aligned sequence reads from a Field Programmable Gate Array (FPGA) device, wherein the FPGA includes one or more configurable digital logic gates that have been configured as a mapping and alignment unit to perform read mapping and alignment.

47. The computer-readable medium of embodiment 36,

wherein the computer is configured to access the one or more memory devices using one or more wired or wireless networks,
wherein a Field Programmable Gate Array (FPGA) device and the one or more memory devices are housed in an expansion card that has been coupled to a circuit board of a sequencer,
wherein the sequencer is configured to generate sequence reads based on an input sample and store the generated sequence reads in the one or more memory devices, and
wherein the mapping and alignment unit of the FPGA is configured to access the one or more memory devices to obtain the generated sequence reads.

48. The computer-readable medium of embodiment 36,

wherein the computer and the sequencer are each configured to access the one or more memory devices using one or more wired or wireless networks,
wherein the Field Programmable Gate Array (FPGA) device and the one or more memory devices are housed in an expansion card that has been coupled to a circuit board of server that is located remotely from the computer and the sequencer,
wherein the sequencer is configured to generate sequence reads based on an input sample, provide the generated sequence reads to the server using the one or more wired or wireless networks for storage, of the generated sequence reads, in the one or more memory devices, and
wherein the mapping and alignment unit of the FPGA is configured to access the one or more memory devices to obtain the generated sequence reads.

**Claims**

1. A method for determining whether a candidate variant allele is not a true variant allele, the method implemented by one or more computer systems and comprising:

obtaining a pileup of reads aligned to a reference sequence;
identifying a plurality of alt-alleles that correspond to the candidate variant allele, wherein each alt-allele corresponds to a base call of at least one read strand in the pileup of reads at a particular reference location of the reference sequence;
obtaining first data indicating whether each of the plurality of alt-alleles occurs in a single read strand of a read having a read orientation in the forward aligned direction;
obtaining second data indicating a location of the plurality of alt-alleles relative to a 5' end of each respective read strand that includes the alt-allele;
obtaining third data indicating a base quality score for each of the reads having a read strand that includes the alt-allele;
providing the first data, the second data, and the third data as an input to a sequencing error probability model;
obtaining output data generated by the sequencing error probability model, wherein the obtained output data indicates a likelihood as to whether the candidate variant allele is a true variant; and
determining, based on the obtained output data, whether the candidate variant allele is a true variant.

2. The method of claim 1, the method further comprising:
based on determining that the candidate variant allele is not a true variant allele:

discarding the obtained output data without adding any additional information to a variant call format (VCF) file that identifies the candidate variant allele; and
storing the VCF without data that identifies the candidate variant allele file in a memory device of a computer system of the one or more computer systems.

3. The method of claim 1, the method further comprising:
based on determining that the candidate variant allele is likely a true variant allele:
adding information to a VCF file that identifies the candidate variant allele; and storing the VCF file that includes information identifying the candidate variant allele in a memory device of a computer system of the one or more computer systems.

4. The method of claim 3, wherein the information added to the VCF file for the candidate variant allele comprises one or more of a position of the candidate variant allele, an identifier of the candidate variant allele, a genotype for the candidate variant allele, or data indicating the likelihood that the candidate variant allele is a true variant.

5. The method of claim 1, wherein the output data indicating a likelihood as to whether the candidate variant allele is a true variant is a value corresponding to a probability indicating whether the candidate variant allele is a true variant.

6. The method of claim 5, wherein determining, based on the obtained output data, whether the candidate variant allele is a true variant comprises:
comparing the probability indicating whether the candidate variant allele is a true variant to a predetermined threshold.

7. The method of claim 6, the method further comprising:

determining, based on the comparison, that the probability indicating whether the candidate variant allele is a true variant does not satisfy the predetermined threshold; and
based on determining that the probability indicating whether the candidate variant allele is a true variant does not satisfy the predetermined threshold, determining that the candidate variant allele is not a true variant

8. The method of claim 6, the method further comprising:

determining, based on the comparison, that the probability indicating whether the candidate variant allele is a true variant satisfies the predetermined threshold; and
based on determining that the probability indicating whether the candidate variant allele is a true variant satisfies the predetermined threshold, determining that the candidate variant allele is likely a true variant.

9. A system comprising:

one or more computers; and
one or more memory devices storing instructions that, when executed by the one or more computers, cause the one or more computers to perform the operations of method claims 1-8.

10. One or more computer readable storage media storing instructions that, when executed by one or more computers, cause the one or more computers to perform the operations of claims 1-8.

FIG. 1

**200**

ACCESS A PILEUP OF ALIGNED SEQUENCE READS
STORED IN ONE OR MORE MEMORY DEVICES

210

OBTAIN DATA REPRESENTING ONE OR MORE
CHARACTERISTICS ASSOCIATED WITH READS
OF THE PILEUP IN A FIRST POSITION

220

PROVIDE ONE OR MORE INPUTS TO A PROBABILITY
MODEL DESCRIBING THE ONE OR MORE
CHARACTERISTICS ASSOCIATED WITH THE READS OF THE
PILEUP AT THE FIRST POSITION

230

OBTAIN OUTPUT DATA GENERATED BY THE
PROBABILITY MODEL, FOR EACH OF A PLURALITY
OF HYPOTHESES BASED ON THE ONE OR MORE INPUTS

240

DETERMINE, BASED ON THE OBTAINED OUTPUT DATA
GENERATED BY THE PROBABILITY MODEL FOR EACH OF
THE PLURALITY OF HYPOTHESES, A LIKELIHOOD THAT A
TRUE VARIANT EXISTS AT THE FIRST POSITION

250

**FIG. 2**

**300**

| ACCESS A PILEUP OF ALIGNED SEQUENCE READS STORED IN ONE OR MORE MEMORY DEVICES |
|---|
310

| OBTAIN INFORMATION REPRESENTING (I) A MAPPING CONFIDENCE SCORE FOR EACH OF THE READS AND (II) A READ-ALLELE SCORE FOR EACH OF THE READS |
|---|
320

| PROVIDE ONE OR MORE INPUTS TO A PROBABILITY MODEL DESCRIBING THE OBTAINED INFORMATION REPRESENTING (I) A MAPPING CONFIDENCE SCORE FOR EACH OF THE READS AND (II) A READ-ALLELE SCORE FOR EACH OF THE READS |
|---|
330

| OBTAIN OUTPUT DATA GENERATED BY THE PROBABILITY MODEL, FOR EACH OF A PLURALITY OF HYPOTHESES BASED ON THE ONE OR MORE INPUTS |
|---|
340

| DETERMINE, BASED ON THE OBTAINED OUTPUT DATA GENERATED BY THE PROBABILITY MODEL FOR EACH OF THE PLURALITY OF HYPOTHESES, A LIKELIHOOD THAT A TRUE VARIANT EXISTS AT THE FIRST POSITION |
|---|
350

**FIG. 3**

**FIG. 4**

EP 4 513 497 A2

**500**

```
510   ┌──────────────────────────────────────────────────────────┐
      │           ACCESS A PILEUP OF ALIGNED SEQUENCE READS      │
      │           STORED IN ONE OR MORE MEMORY DEVICES           │
      └──────────────────────────────────────────────────────────┘
```

ACCESS A PILEUP OF ALIGNED SEQUENCE READS STORED IN ONE OR MORE MEMORY DEVICES

OBTAIN INFORMATION DESCRIBING (I) A READ ORIENTATION FOR EACH OF THE READS, (II) A POSITION OF EACH BASE WITH REFERENCE TO THE 5′ END OF THE READ, (III) A READ-ALLELE SCORE FOR EACH OF THE READS, AND (IV) A BASE QUALITY SCORE FOR EACH READ

PROVIDE ONE OR MORE INPUTS TO A PROBABILITY MODEL DESCRIBING THE OBTAINED INFORMATION DESCRIBING (I) A READ ORIENTATION FOR EACH OF THE READS, (II) A POSITION OF EACH BASE WITH REFERENCE TO THE 5′ END OF THE READ, (III) A READ-ALLELE SCORE FOR EACH OF THE READS, AND (IV) A BASE QUALITY SCORE FOR EACH READ

OBTAIN OUTPUT DATA GENERATED BY THE PROBABILITY MODEL, FOR EACH OF A PLURALITY OF HYPOTHESES BASED ON THE ONE OR MORE INPUTS

DETERMINE, BASED ON THE OBTAINED OUTPUT DATA GENERATED BY THE PROBABILITY MODEL FOR EACH OF THE PLURALITY OF HYPOTHESES, A LIKELIHOOD THAT A TRUE VARIANT EXISTS AT THE FIRST POSITION

**FIG. 5**

600

610 ACCESS A PILEUP OF ALIGNED SEQUENCE READS
STORED IN ONE OR MORE MEMORY DEVICES

620 OBTAIN INFORMATION REPRESENTING (I) A READ
ORIENTATION FOR EACH OF THE READS, (II) A POSITION OF
EACH BASE WITH REFERENCE TO THE 5′ END OF THE READ,
(III) A MAPPING CONFIDENCE SCORE FOR EACH OF THE
READS AND, (IV) A READ-ALLELE SCORE FOR EACH OF THE
READS, AND (V) A BASE QUALITY SCORE FOR EACH READ

630 PROVIDE ONE OR MORE INPUTS TO A
PROBABILITY MODEL DESCRIBING THE OBTAINED
INFORMATION REPRESENTING (I) A READ ORIENTATION
FOR EACH OF THE READS, (II) A POSITION OF EACH BASE
WITH REFERENCE TO THE 5′ END OF THE READ, (III) A
MAPPING CONFIDENCE SCORE FOR EACH OF THE READS
AND, (IV) A READ-ALLELE SCORE FOR EACH OF THE READS,
AND (V) A BASE QUALITY SCORE FOR EACH READ

640 OBTAIN OUTPUT DATA GENERATED BY THE
PROBABILITY MODEL, FOR EACH OF A PLURALITY
OF HYPOTHESES BASED ON THE ONE OR MORE INPUTS

650 DETERMINE, BASED ON THE OBTAINED OUTPUT DATA
GENERATED BY THE PROBABILITY MODEL FOR EACH OF
THE PLURALITY OF HYPOTHESES, A LIKELIHOOD THAT A
TRUE VARIANT EXISTS AT THE FIRST POSITION

**FIG. 6**

700

710 — RECEIVE, BY A COMPUTER STORING A PROBABILITY MODEL, INPUT DATA THAT INCLDUES ONE OR MORE OF INFORMATION REPRESENTING (I) A READ ORIENTATION FOR EACH OF THE READS, (II) A POSITION OF EACH BASE WITH REFERENCE TO THE 5′ END OF THE READ, (III) A MAPPING QUALITY SCORE FOR EACH OF THE READS, (IV) A READ-ALLELE SCORE FOR EACH OF THE READS, (V) A BASE QUALITY SCORE FOR EACH READ

720 — DETERMINE, BY THE COMPUTER, A SET OF ONE OR MORE HYPOTHESES BASED ON THE RECEIVED INPUT DATA

730 — DETERMINE, BY THE COMPTUER AND USING THE PROBABILITY MODEL, A SCORE FOR EACH OF THE ONE OR MORE HYPOTHESES THAT INDICATES A PROBABILITY THAT EACH RESPECTIVE HYPOTHESIS IS TRUE

740 — PROVIDE, BY THE COMPUTER, OUTPUT DATA GENERATED BY THE PROBABILITY MODEL THAT INCLUDES THE SCORE FOR EACH OF THE ONE OR MORE HYPOTHESES

FIG. 7

**FIG. 8**

**FIG. 9A**

1 : 54110087

G |F|E    Ph(G)  +Ph(F,E)  +Ph(R|G')  = Ph(R,G'),  norm'd

00 |-|-:  -0.00 +   0.00 +  610.32 =  610.32, 480.46, PL by strand: 279.39, 330.93

10 |-|-:  34.77 +   0.00 +   95.09 =  129.86,   -0.00, PL by strand:   41.96, 53.13

11 |-|-:  37.77 +   0.00 +  656.32 =  694.09, 564.23, PL by strand: 296.39, 359.93

00 |1|-:  -0.00 +  84.77 +   95.09 =  179.86,   50.00, beta = 0.5000

11 |0|-:  37.77 +  50.00 +   95.09 =  182.86,   53.00, beta = 0.5000

00 |-|1:  -0.00 + 173.46 +   83.05 =  256.51, 126.65, n0=11, q_mean=36.09, Ph (E)=90.23, score=123.16; n1=17, q_mean=33.29, Ph(E)=83.24, score=133.35;

11 |-|0:  37.77 + 174.92 +   95.09 =  307.78, 177.92, n0=14, q_mean=36.36, Ph (E)=90.89, score=132.86; n1=18, q_mean=33.61, Ph(E)=84.03, score=137.15;

Genotyping Matrix (phred scale):
    50.0001         NaN
     0.0001      53.0001

**FIG. 9B**

EP 4 513 497 A2

FIG. 10A

1 : 8959903

$\underline{G \ |F|E} \quad \underline{Ph(G)} + \underline{Ph(F,E)} + \underline{Ph(R|G')} = \underline{Ph(R,G'), \ norm'd}$

00 |-|-: -0.00 + 0.00 + 259.62 = 259.62, 108.30, PL by strand: 131.31, 128.31

10 |-|-: 34.77 + 0.00 + 116.55 = 151.32, -0.00, PL by strand: 77.43, 39.12

11 |-|-: 37.77 + 0.00 + 1335.41 = 1373.18, 1221.86, PL by strand: 974.71, 360.70

00 |1|-: -0.00 + 80.42 + 72.54 = 152.96, 1.65, beta = 0.1538

11 |0|-: 37.77 + 50.00 + 116.55 = 204.32, 53.00, beta = 0.5000

00 |-|1: -0.00 + 171.33 + 74.41 = 245.74, 94.42, n0=14, q_mean=35.71, Ph (E)=89.29, score=130.60; n1=11, q_mean=32.82, Ph(E)=82.05, score=115.14;

11 |-|0: 37.77 + 166.54 + 116.55 = 320.86, 169.54, n0=26, q_mean=37.77, Ph (E)=84.42, score=161.85; n1=13, q_mean=32.85, Ph(E)=82.12, score=121.23;

Genotyping Matrix (phred scale):

3.9117      NaN

2.2642      55.2642

1080

1090

**FIG. 10B**

EP 4 513 497 A2

**FIG. 11A**

1 : 214459427

```
     G |F|E    Ph(G) +Ph(F,E) +Ph(R|G') = Ph(R,G'),  norm'd
1161 00|-|-:  -0.00 +   0.00 +  224.01 =  224.01,  160.51,  PL by strand:  224.01,   -0.00
1162 10|-|-:  34.77 +   0.00 +  115.72 =  150.49,   87.00,  PL by strand:   64.69,   51.03
1163 11|-|-:  37.77 +   0.00 +  931.79 =  969.56,  906.06,  PL by strand:  240.70,  691.09
1164 00|1|-:  -0.00 +  84.77 +  102.83 =  187.60,  124.11,  beta = 0.3250
1165 11|0|-:  37.77 +  50.00 +  115.72 =  203.49,  140.00,  beta = 0.5000
1166 00|-|1:  -0.00 +  16.71 +   46.79 =   63.50,   -0.00,  n0=19, q_mean=10.68, Ph (E)=16.71, score= 63.50; n1= 0, q_mean= 0.00, Ph(E)= 0.00, score= 0.00;
1167 11|-|0:  37.77 + 126.11 +  115.72 =  279.60,  216.10,  n0=25, q_mean=14.56, Ph (E)=36.40, score=101.09; n1=17, q_mean=35.88, Ph(E)= 89.71, score=140.73;
     Genotyping Matrix (phred scale):
         0.0000      NaN
        86.9952   139.9952
```

1180    1190

**FIG. 11B**

EP 4 513 497 A2

**FIG. 12A**

1260

1 : 53545723

G |F|E    Ph(G) +Ph(F,E) +Ph(R|G') = Ph(R,G'), norm'd

1261  00 | - | - :  -0.00 +   0.00 +  199.55 =  199.55, 140.45, PL by strand:  81.16, 118.39
1262  10 | - | - :  34.77 +   0.00 +   76.67 =  111.44,  52.34, PL by strand:  42.46,  34.21
1263  11 | - | - :  37.77 +   0.00 +  300.78 =  338.55, 279.45, PL by strand: 236.16,  64.62
1264  00 | 1 | - :  -0.00 +  84.77 +   76.67 =  161.44, 102.34, beta = 0.5000
1265  11 | 0 | - :  37.77 +  50.00 +   76.72 =  164.49, 105.39, beta = 0.4828
1266  00 | - | 1 :  -0.00 +   3.27 +   55.83 =   59.10,   -0.00 n0= 9, q_mean= 5.00, Ph (E)=   0.00, score=21.63; n1=13, q_mean= 9.31, Ph(E)= 3.27, score= 37.48;
1267  11 | - | 0 :  37.77 +  49.16 +   82.40 =  169.33, 110.22, n0=16, q_mean=15.06, Ph (E)=37.66, score=80.12; n1= 5, q_mean= 4.60, Ph(E)=11.50, score= 51.44;

Genotyping Matrix (phred scale):

    0.0000        NaN        1280          1290
   52.3365     105.3922

**FIG. 12B**

EP 4 513 497 A2

**EP 4 513 497 A2**

**REFERENCES CITED IN THE DESCRIPTION**

**Patent documents cited in the description**

- US 62710348
- US 20160306922 A